# EUROPEAN PATENT SPECIFICATION

(11) **EP 4 359 391 B1**
(45) Date of publication and mention of the grant of the patent: **18.03.2026**
(21) Application number: 22738012.8
(22) Date of filing: 23.06.2022
(51) Int. Cl.: C07D 251/46, C07D 495/04, C07D 401/06, C07D 401/14, C07D 403/12, A61P 25/00, A61P 31/00, A61P 35/00, A61K 31/53

(54) **TRIAZINE DERIVATIVES FOR THE TREATMENT OF TUMOURS AND NEURODEGENERATIVE DISORDERS**
TRIAZINDERIVATE ZUR BEHANDLUNG VON TUMOREN UND NEURODEGENERATIVEN ERKRANKUNGEN
DÉRIVÉS DE TRIAZINE POUR LE TRAITEMENT DE TUMEURS ET DE TROUBLES NEURODÉGÉNÉRATIFS

(30) Priority: 25.06.2021 IT 202100016775
(43) Date of publication of application: 01.05.2024
(73) Proprietor: UNIVERSITA' DEGLI STUDI DI MILANO, 20122 Milano (IT)
(72) Inventor: MARINELLI, Luciana, 80138 NAPOLI (IT); AROSIO, Daniela, 20122 MILANO (IT); SENECI, Pierfausto, 20122 MILANO (IT)
(74) Representative: Studio Torta S.p.A.
(86) International application number: PCT/IB2022/055828
(87) International publication number: WO 2022/269532

(56) References cited:
- EP-A1- 3 669 872
- WO-A1-2015/034820
- WO-A1-2018/195321
- WO-A1-2020/244518
- WO-A1-2020/255021
- CN-A- 111 559 981
- YANWEN ZHONG ET AL: "The Characteristics of PD-L1 Inhibitors, from Peptides to Small Molecules", MOLECULES, vol. 24, no. 10, 1 January 2019 (2019-01-01), DE, pages 1940, XP055668971, ISSN: 1433-1373, DOI: 10.3390/molecules24101940
- RUSSOMANNO PASQUALE ET AL: "Interfering with the Tumor-Immune Interface: Making Way for Triazine-Based Small Molecules as Novel PD-L1 Inhibitors", JOURNAL OF MEDICINAL CHEMISTRY, vol. 64, no. 21, 20 October 2021 (2021-10-20), US, pages 16020 - 16045, XP055955560, ISSN: 0022-2623, DOI: 10.1021/acs.jmedchem.1c01409

## Description

### Technical Field of the Invention

The present invention relates to triazine derivatives capable of inhibiting PD-1/PD-L1 binding for use in the treatment, prevention and diagnosis of tumours, bacterial and/or viral infections and neurodegenerative disorders.

### State of the Art

Rudolf Virchow, who observed the prevalence of leucocytes in tumours, more than a century ago proposed for the first time the existence of an intimate relationship between cancer and immune function. Since then and for at least the next 100 years, there has been limited progress in understanding the biological pathways activated by the interaction between tumour cells and the immune system.

Although much remains to be understood, it is now clear that tumour cells manipulate the immune system so as not to attack malignant cells and that this is achieved through multiple mechanisms such as immunosuppressive cytokines or so-called Immune Checkpoint Receptors (ICRs). Both of the above molecular mechanisms contribute to the local remodelling of the tumour microenvironment (TME) and in secondary organs they provide "premetastatic niches", where a fertile soil for immune escape and cancer growth is guaranteed.

Among the ICRs, CTLA-4 (cytotoxic T-lymphocyte protein 4), PD-1 (Programmed cell Death protein 1), IDO (Indoleamine 2, 3-dioxygenase), TIM-3 (T cell immunoglobulin and mucin domain-containing protein 3) and LAG-3 (Lymphocyteactivation gene 3) have received the most attention so far.

In particular, PD-1 is a cell surface receptor expressed by CD8+ T cells upon activation, during priming or expansion. It is now known that TME can induce overexpression of the PD-1 receptor on infiltrated T cells, while its physiological ligand PD-L1 is overexpressed on tumour cell membranes. The recognition and binding of PD-1/PD-L1 generates an inhibitory signal that attenuates T-cell activity in cancer patients, thereby inhibiting anti-tumour immunity and causing T cell depletion. Effector T-cells (Teff) depletion has been found to be an important negative feedback loop that ensures immune homoeostasis against cancer. In this perspective, the PD-1/PD-L1 axis can be compromised targeting PD-1 or PD-L1 with antibodies.

Two PD-1-specific mAbs, Pembrolizumab (Merck's Keytruda) and Nivolumab (Bristol-Myers Squibb's Opdivo) were among the first clinical trials that cancer can be treated through modulation of the immune response. Following this success, PD-L1-specific antibodies (Atezolizumab, Durvalumab, Avelumab) were also studied.

Currently, anti-PD-1/anti-PD-L1 antibodies have been tested in more than 1,000 clinical trials and approved for the treatment of several cancer types, including melanoma, renal cell carcinoma, Hodgkin's lymphoma, bladder cancer, head and neck squamous cell carcinoma (HNSCC), and more recently non-small cell lung carcinoma (NSCLC).

However, despite their remarkable success in patients with particular tumour types, antibodies have well-known disadvantages when used as therapeutic agents, including, but not limited to, high production costs, insufficient oral bioavailability, long circulating half-life, poor tissue and tumour penetration capacity, and immune-related adverse events.

In an attempt to overcome some of these problems, a number of small molecules, such as macrocyclic peptides, and organic compounds targeting PD-L1 have been studied.

WO 2015/160641 protects a number of dibenzyl etherbased compounds, e.g. the compound known as BMS-202, capable of disrupting the PD-1/PD-L1 complex with an IC50 ranging from 1 to 300 nM.

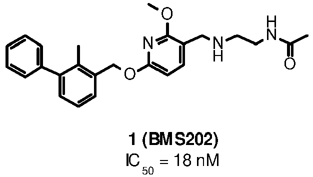

Only in 2015, the structural basis of the human PD-1/PD-L1 protein-protein interaction (PPI) was identified by X-ray crystallography.

Subsequently, structures of PD-L1 complexed with antibodies, peptide macrocycles and small organic compounds were also described, revealing that ligands can recognise partially overlapping regions on the surface of PD-L1.

However, the flat and hydrophobic binding surface of PD-L1 made it immediately clear that the rational design of small inhibitors is very complex. In this scenario, the discovery of compound BMS202 represented a valuable starting point for the design of PD-L1 ligands that led to the synthesis of compounds A-E reported below.

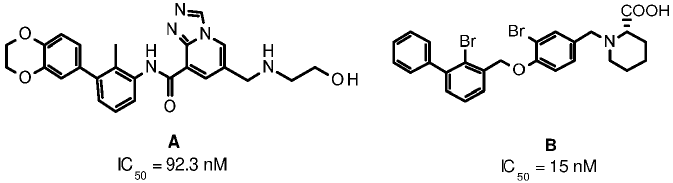

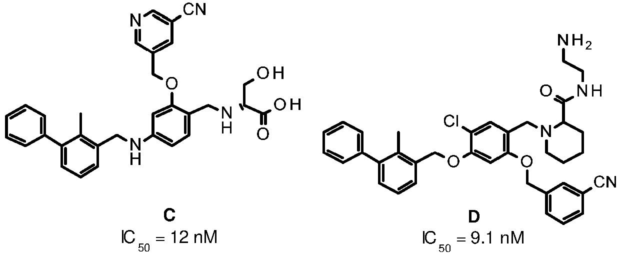

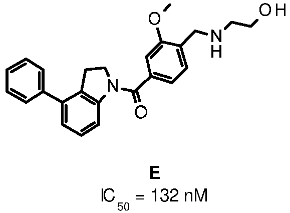

Therefore, a number of studies have been carried out that aim to evaluate the in vivo anticancer properties of biphenyl-based compounds. Although some studies are of doubtful value with regard to PD-L1-dependent effects in mice, other studies seem to be fully significant and promising in this respect. For example, compounds C and D were tested in a humanised mouse model with an immune checkpoint demonstrating to be highly effective in suppressing tumour growth, thereby pushing for further development of biphenyl-based compounds. Other PD1/PD-L1 inhibitors are disclosed in EP3669872, WO2015034820, Yanwen Zhong et al (Molecules, vo. 24, n. 10, 2019, page 1940), WO2020255021, WO2020244518, WO2018195321 and CN111559981.

The development of structurally new small PD-L1 ligands would therefore be of utmost importance for a full understanding of the potential of small PD-1/PD-L1 inhibitors.

There is therefore a need for alternative treatments that act on the PD-1/PD-L1 interaction and that are free of the disadvantages of treatments known in the art.

### Subject and Summary of the Invention

Aim of the present invention is therefore to provide small-sized compounds capable of inhibiting PD-1/PD-L1 binding that are free of the disadvantages of the prior art.

This aim is achieved by a compound according to claim 1, a pharmaceutical composition thereof according to claim 2, and a use thereof according to claims 3 and 4.

Starting from the compounds of the prior art, knowing that the biphenyl ether moiety is the driving group for the surface binding of PD-L1 and that an aromatic core is required to oppositely orient the two main PD-L1-interacting chains (e.g., the 1,4-diamino-acetyl and biphenyl etherbased chains in compound BMS202), the inventors sought an accessible and synthetically flexible ring substitution.

Surprisingly, the triazines of the invention proved to be particularly efficient in selectively binding PD-L1 despite the fact that their electrochemical properties are substantially different from those of the ring of pyridine (BMS202) and benzene (core of compounds A-E), and the relative orientation of the substituents is therefore similar but not completely superimposable to that of the compounds of the prior art (para orientation in compounds BMS202 and compounds B-E, meta orientation in the molecules of the invention). In particular, the triazines of the invention were tested by means of a time-resolved homogeneous fluorescence binding (HTRF) assay, which provided an IC50 for each compound found to be active by NMR.

Compound 4 was identified as a lead compound capable of specifically binding PD-L1 and not PD-1.

### Brief Description of the Drawings

The present invention will now be described in detail with reference to the figures in the accompanying drawings, wherein:
- Figure 1 shows the 1D ¹H NMR spectra of PDL1 (10 µM) in the absence (panel a), and in the presence of BMS-202 (panel b, dashed) and 10 (panel c, dashed).
- Figure 2 shows the predicted docking binding position for compound 4 at the PD-L1 homodimer binding site.
- Figure 3 shows the side view (A) and bottom view (B) of the overlap between the predicted docking position for compound 4 and the X-ray structure of BMS202 at the homodimeric PD-L1 binding site.
- Figure 4 shows the differential scanning calorimetry (DSC) profiles for PD-L1 in the absence (dotted line) and in the presence of 1 molar equivalent of compound 4 (dotted line) or compound BMS-202 (dotted line), recorded at 0.5°C/min heating.

### Detailed Description of Preferred Embodiments of the Invention

The following paragraphs provide the chemical characteristics of the compounds according to the invention and are intended to apply uniformly throughout the description and to all claims unless a definition is expressly stated that provides a broader definition.

The term "alkyl", as used herein, refers to saturated aliphatic hydrocarbons. This term includes linear (unbranched) or branched chains.

Non-limiting examples of alkyl groups according to the invention are, for example, methyl, ethyl, propyl, isopropyl, n-butyl, isobutyl, tert-butyl, n-pentyl, isopentyl, n-hexyl and the like.

The term "cycloalkyl" as used herein, refers to a saturated or partially unsaturated carbocyclic group having a single ring. It includes cycloalkenyl groups.

Non-limiting examples of cycloalkyl groups according to the invention are, for example, cyclopropane, cyclobutane, cyclopentane, cyclohexane, cycloheptane, cyclopentene, cyclohexene, cyclohexadiene and the like.

The term "heterocycloalkyl" group, ("non-aromatic heterocyclic" group), refers to a cycloalkyl group (non-aromatic group) wherein at least one of the carbon atoms has been substituted with a heteroatom selected from nitrogen, oxygen and sulphur. Heterocycloalkyl groups may be unsubstituted or substituted with one or more substituents as defined below.

Examples of heterocycloalkyls include, but are not limited to, lactams, lactones, cyclic imides, cyclic thioimides, cyclic carbamates, 1-(1,2,5,6-tetrahydropyridyl), tetrahydrothiopyran, 4H-pyran, tetrahydropyran, piperidine (2-piperidinyl, 3-piperidinyl), 1,3-dioxin, 1,3-dioxane, 1,4-dioxin, 1,4-dioxane, piperazine, 1,3-oxathiane, 1,4-oxathine, 1,4-oxathiane, tetrahydro-1,4-thiazine, 2H-1,2-oxazine, morpholine (4-morpholinyl 3-morpholinyl, 2-morpholinyl) trioxane, hexahydro-1,3,5-triazine, tetrahydrothiophene, tetrahydrofuran (tetrahydrofuran-2-yl, tetrahydrofuran-3-yl), pyrroline, pyrrolidine, pyrrolidone, pyrrolidinedione, pyrazoline pyrazolidine, imidazoline, imidazolidine, 1,3-dioxol, 1,3-dioxolane, 1,3-dithiol, 1,3-dithiolane, isoxazoline, isoxazolidine, oxazoline, oxazolidine, oxazolidinone, thiazoline, thiazolidine, and 1,3-oxathiolane.

The term "aryl", as used herein, refers to a hydrocarbon consisting of a monocarbocyclic, bicarbocyclic or tricarbocyclic cyclic system wherein the rings are fused together and at least one of the carbocyclic rings is aromatic. The term "aryl" refers for example to a cyclic aromatic such as a 6-membered hydrocarbon ring, two sixmembered fused hydrocarbon rings. Non-limiting examples of aryl groups are, for example, phenyl, alpha- or betanaphthyl, 9,10-dihydroanthracenyl, indanyl, fluorenyl and the like.

Unless otherwise indicated, the term "substituted", as used herein, means that one or more hydrogen atoms of the aforementioned groups are substituted with another nonhydrogen atom, or a functional group, provided that the normal valences are maintained and that the substitution results in a stable compound.

The persons skilled in the technique of organic chemistry will appreciate that many organic compounds can form complexes with solvents in which they are reacted or from which they are precipitated or crystallise. These complexes are known as "solvates". For example, a complex with water is known as a "hydrate". Solvates of the compounds of the invention fall within the scope of protection of the invention. The compounds of the invention can be easily isolated in association with solvent molecules by crystallisation or evaporation of an appropriate solvent to deliver the corresponding solvates.

The compounds of the invention can be in crystalline form. In some embodiments, the crystalline forms of the compounds of the invention are polymorphic.

The present invention also includes isotopically labelled compounds, which are identical to those listed in claim 1, but differ in that one or more atoms are substituted with an atom having an atomic mass or mass number that differs from the atomic mass or mass number usually found in nature. Examples of isotopes that may be incorporated into the compounds of the invention include hydrogen, carbon, nitrogen, and oxygen isotopes such as ²H, ³H, ¹¹C ¹³C, ¹⁴C, ¹⁵N, ¹⁷O.

The compounds of the present invention containing the above-mentioned isotopes and/or other isotopes of other atoms fall within the scope of protection of the present invention. The isotopically labelled compounds of the present invention, for example those in which radioactive isotopes such as ³H and ¹⁴C are incorporated, are useful in assays of tissue distribution of drug and/or substrate. Tritiated isotopes, i.e. ³H and carbon-14, i.e. ¹⁴C, are particularly preferred for their ease of preparation and detectability. ¹¹C isotopes are particularly useful in PET (positron emission tomography). In addition, substitution with heavier isotopes such as deuterium, i.e. ²H, may provide certain therapeutic advantages resulting from increased metabolic stability, e.g. increased *in vivo* half-life or reduced dosing requirements, and may therefore be referred to in certain circumstances. The isotopically labelled compounds of the present invention of the present invention can generally be prepared by performing the procedures described in the diagrams and/or in the following examples, replacing a non-isotopically labelled reagent with an easily available isotopically labelled reagent.

Certain groups/substituents included in the present invention may be present as isomers. Consequently, in some embodiments, the compounds of the present invention may have axial asymmetries and, correspondingly, may exist in the form of optical isomers such as one form (R), one form (S) and the like. The present invention includes within the scope of protection all such isomers, including racemates, enantiomers and mixtures thereof.

In particular, the scope of protection of the present invention includes all stereoisomeric forms, including enantiomers, diastereoisomers and mixtures thereof, including racemates, and the general reference to the compounds of the present invention includes all stereoisomeric forms, unless otherwise indicated.

In general, the compounds (if any) that are chemically very unstable, either *per se* or in water, which are clearly unsuitable for pharmaceutical use through all routes of administration, whether oral, parenteral or otherwise, should be considered excluded from the compounds of the invention. Such compounds are known to the skilled chemist.

Finally, the compounds of the present invention can form salts.

According to the present disclosure, the compounds of formula (I) are provided (not part of the invention): 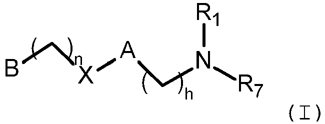 or its pharmaceutically acceptable salts, solvates or isomers wherein:
**R₁** is selected from the group consisting of H and linear or branched C₁-C₄alkyl;
**R₇** is (CₘH₂ₘ-₂)RR'-R₂
wherein **m** is an integer between 0 and 4;
**R** and **R'** are selected independently from the group consisting of H, OH, C₁-C₄alkyl, COOH and COO-C₁-C₄alkyl;
**R₂** is selected from the group consisting of
H,
C₁-C₄alkyl,
C₁-C₆cycloalkyl optionally substituted with OH,
C₁-C₆heterocycloalkyl,
phenyl optionally substituted with NO₂;
OH,
COOH,
COO-C₁-C₄alkyl,
COO-C₁-C₄alkylaryl,
CONH-C₁-C₆alkyl-NH₂,
CONHOH,
CO-C₁-C₆heterocycloalkyl,
NH₂,
NHSO₂-C₁-C₆alkyl,
SO₂NH₂,
SO₂NH-C₁-C₆alkyl,
NHCO-C₁-C₆alkyl optionally substituted with R₆, and 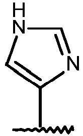
or **R₁** and **R₇** are linked so as to form together with the nitrogen a 5 or 6 atoms ring, optionally comprising another nitrogen atom, oxygen or sulphur, wherein said ring is substituted with one or more R₂;
**A** is a 1,3,5-triazine optionally substituted with R₃;
**R₃** is selected from the group consisting of halogen, linear or branched C₁-C₄alkyl, OH, O-C₁-C₄alkyl, NH-C₁-C₇alkyl-R₆, O-C₁-C₄alkylaryl optionally substituted with a group selected from the group consisting of CN, N₃, CONH₂, COO- C₁-C₄alkyl;
**h** is an integer between 0 and 1;
**X** is selected from the group consisting of O and NH;
**n** is an integer between 1 and 4;
**B** is selected from the group consisting of 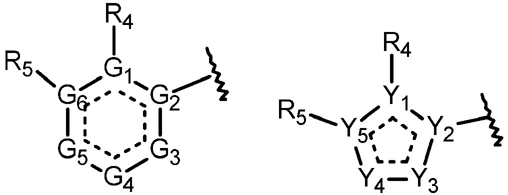
wherein **Y₁, Y₂, Y₃, Y₄, Y₅** are selected independently from the group consisting of CH, S, SH, N, NH and O and at least two of them are CH; and
**G₁, G₂, G₃**, **G₄, G₅** and **G₆** are selected independently from the group consisting of CH, S, SH, N, NH and O;
**R₄** is selected from the group consisting of H, linear or branched C₁-C₄alkyl, halogen, CN, OH;
**R₅** is selected from the group consisting of phenyl, pyridine 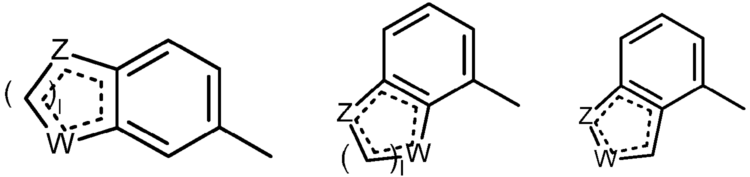 and 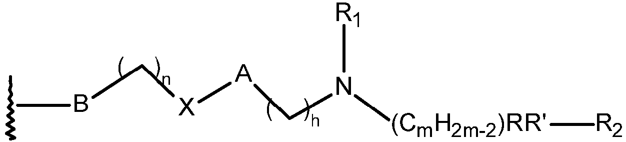
**Z** and **W** are selected independently from the group consisting of S, CH, C, O, N and NH;
**l** is an integer between 1 and 2;
**R₆** is selected from the group consisting of C₂-C₄alkynyl, CONH-C₁-C₆alkyl-NHCO-C₁-C₆alkylSH, 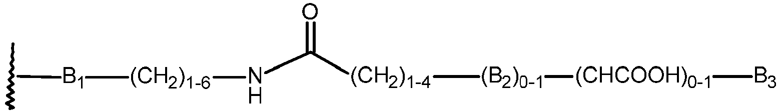
wherein **B₁** is selected from the group consisting of 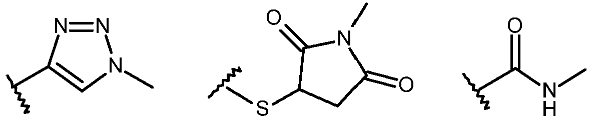
**B₂** is 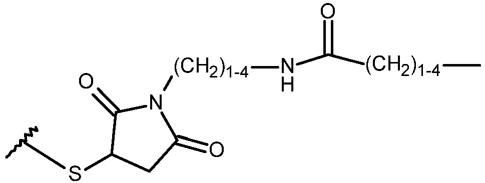
**B₃** is selected from the group consisting of

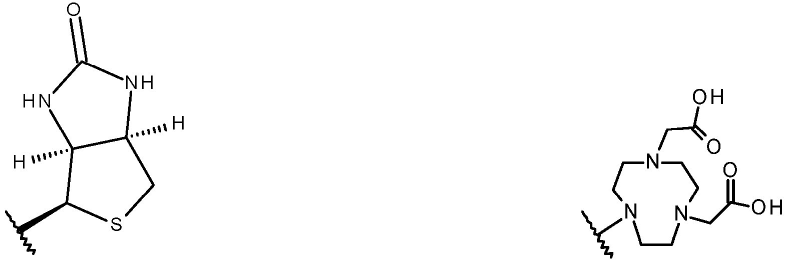

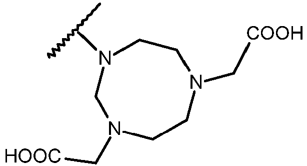

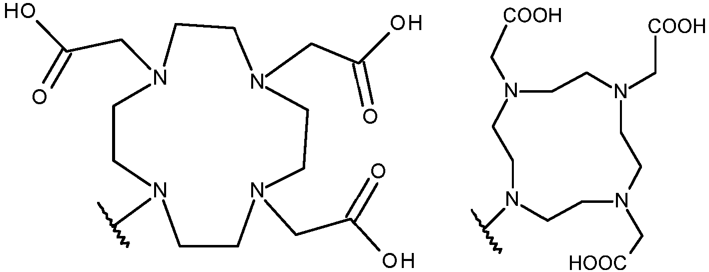

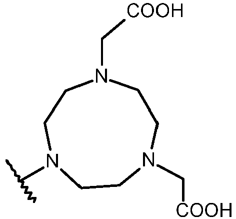

In a first embodiment, the compounds of the present disclosure have formula (II): 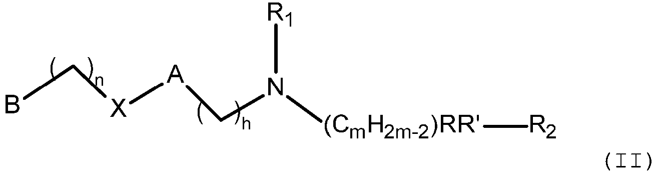 wherein:
**R₁** is selected from the group consisting of H and linear or branched C₁-C₄alkyl;
**m** is an integer between 0 and 4;
**R** and **R'** are selected independently from the group consisting of H, OH, C₁-C₄alkyl, COOH and COO-C₁-C₄alkyl;
**R₂** is selected from the group consisting of
H,
C₁-C₄alkyl,
C₁-C₆cycloalkyl optionally substituted with OH,
C₁-C₆heterocycloalkyl,
phenyl optionally substituted with NO₂;
OH,
COOH,
COO-C₁-C₄alkyl,
COO-C₁-C₄alkylaryl,
CONH-C₁-C₆alkyl-NH₂,
CONHOH,
CO-C₁-C₆heterocycloalkyl,
NH₂,
NHSO₂-C₁-C₆alkyl,
SO₂NH₂,
SO₂NH-C₁-C₆alkyl,
NHCO-C₁-C₆alkyl optionally substituted with R₆, and 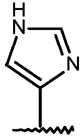
**A** is a 1,3,5-triazine optionally substituted with R₃;
**R₃** is selected from the group consisting of halogen, linear or branched C₁-C₄alkyl, OH, O-C₁-C₄alkyl, NH-C₁-C₇alkyl-R₆, O-C₁-C₄alkylaryl optionally substituted with a group selected from the group consisting of CN, N₃, CONH₂;
**h** is an integer between 0 and 1;
**X** is selected from the group consisting of O and NH;
n is an integer between 1 and 4;
B is selected from the group consisting of 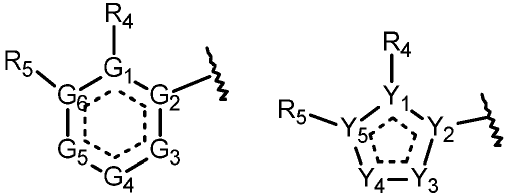
wherein **Y₁, Y₂, Y₃, Y₄, Y₅** are selected independently from the group consisting of CH, S, SH, N, NH and O and at least two of them are CH; and
**G₁, G₂, G₃**, **G₄, G₅** and **G₆** are selected independently from the group consisting of CH, S, SH, N, NH and O;
**R₄** is selected from the group consisting of H, linear or branched C₁-C₄alkyl, halogen, CN, OH;
**R₅** is selected from the group consisting of phenyl, 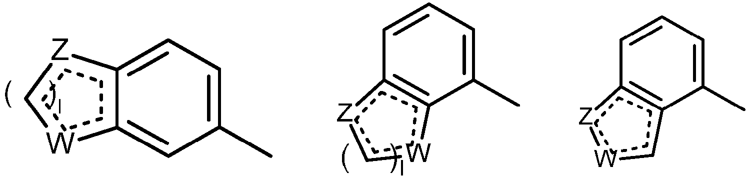 and 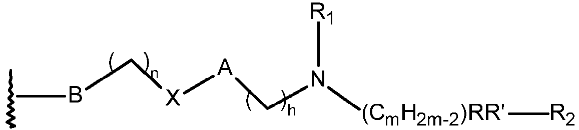
**Z** and **W** are selected independently from the group consisting of S, CH, C, O, N and NH;
**l** is an integer between 1 and 2;
**R₆** is selected from the group consisting of C₂-C₄alkynyl, CONH-C₁-C₆alkyl-NHCO-C₁-C₆alkylSH, 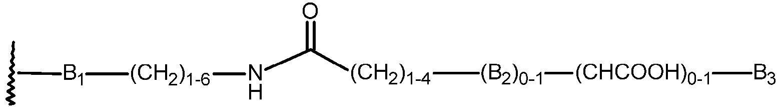
wherein **B₁** is selected from the group consisting of 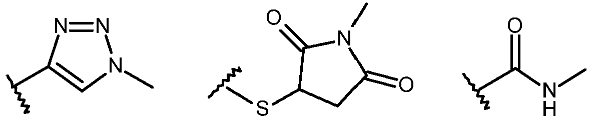
**B₂** is 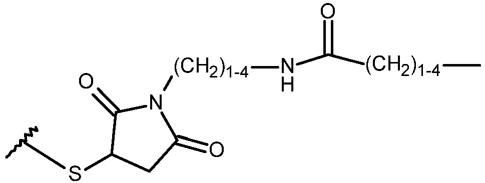
**B₃** is selected from the group consisting of

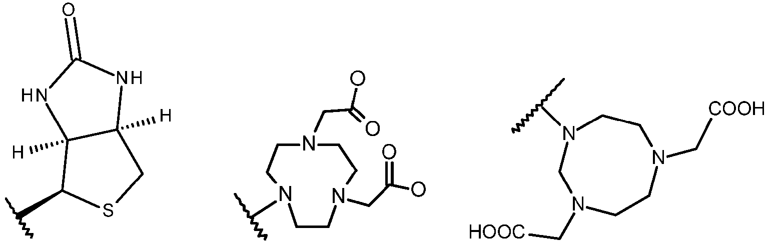

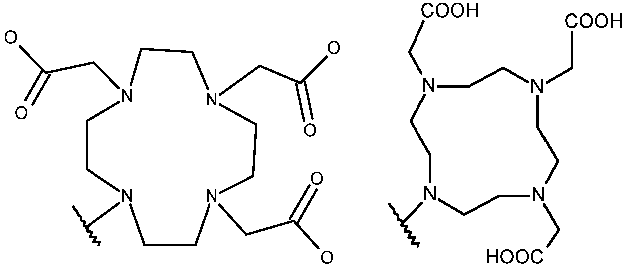

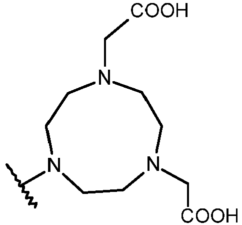

In a second embodiment **R₁** is H.

In a second embodiment, **R₂** is NHCOCH₃, SO₂NH₂, NHSO₂CH₃, NHCOCH₂C≡CH, COOCH₂phenyl, 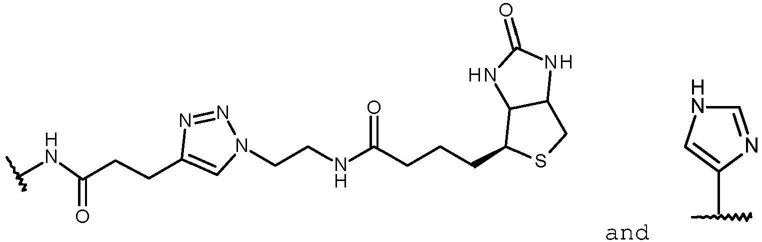 .

In a third embodiment, **R₃** is selected from the group consisting of Cl, CH₃, OH, OCH₃, NHCH₂C≡CH, 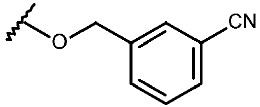 and

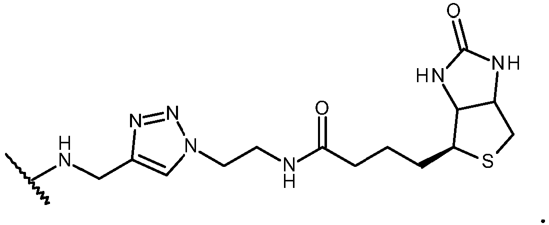

In a further embodiment **R₅** is selected from the group consisting of phenyl, pyridine

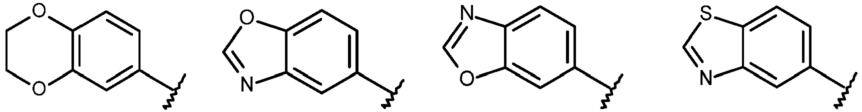

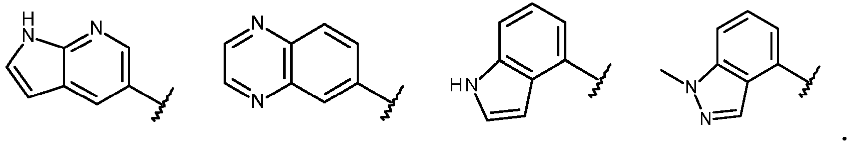

In a further embodiment **R₆** is selected from the group consisting of C₂-C₄alkynyl, CONH(CH₂)₅NHCO(CH₂)₂SH,

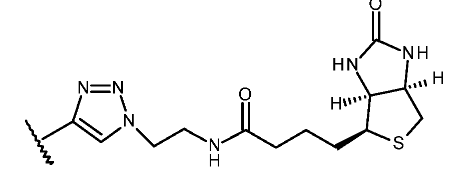

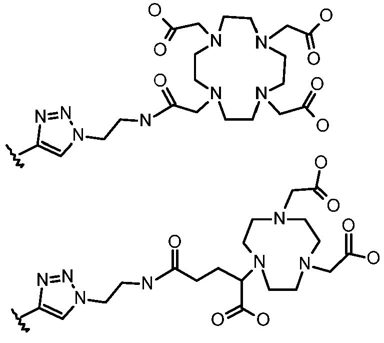

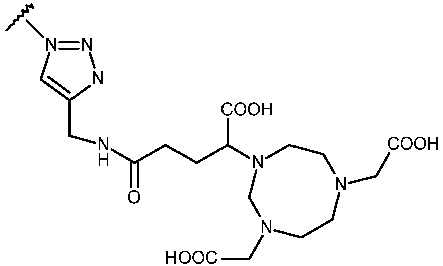

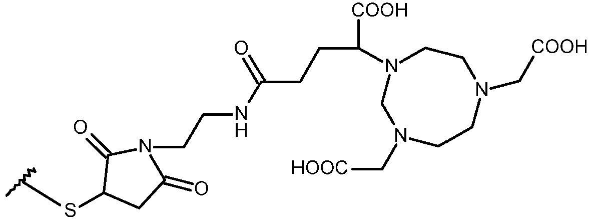

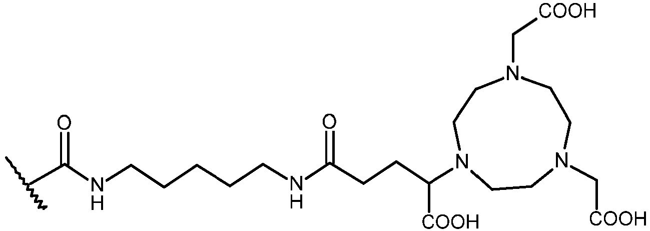

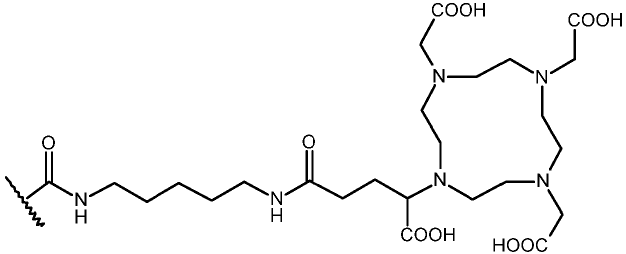

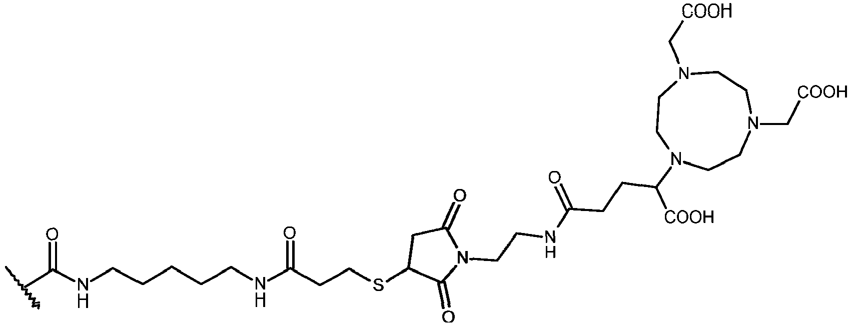

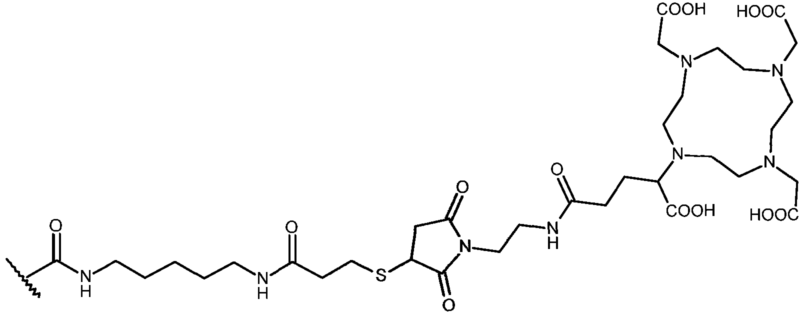

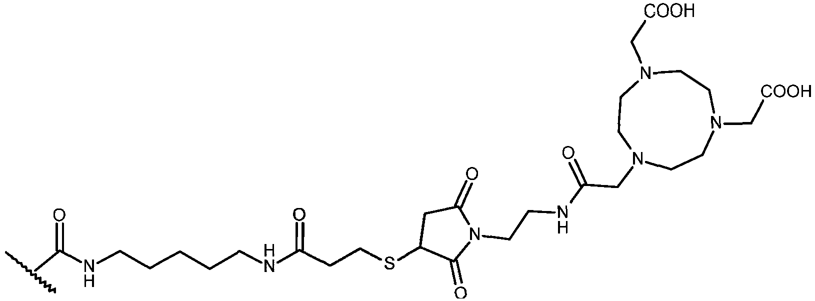

According a first aspect of the invention, the compounds of the present invention are the following compounds with the exception of compounds 58-65:

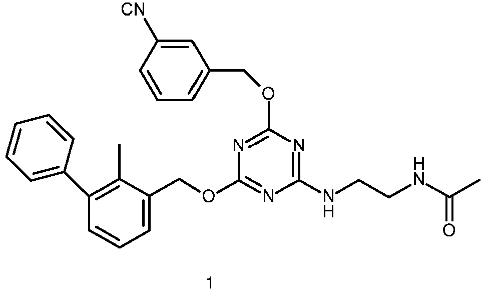

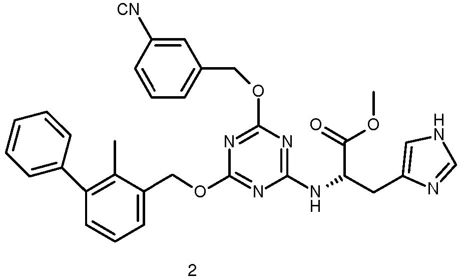

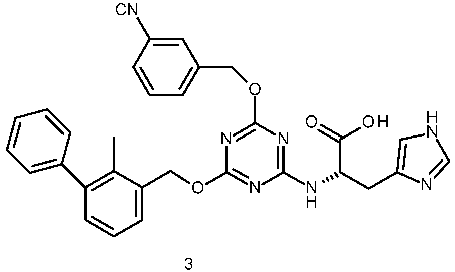

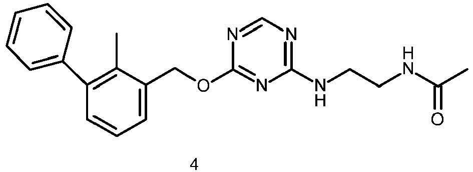

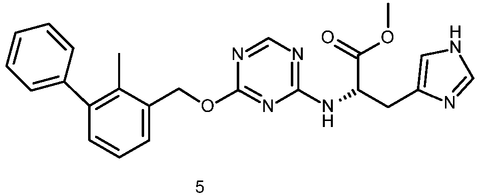

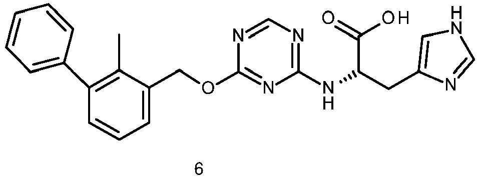

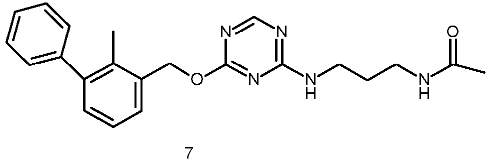

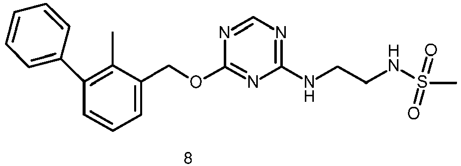

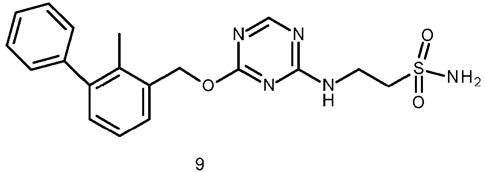

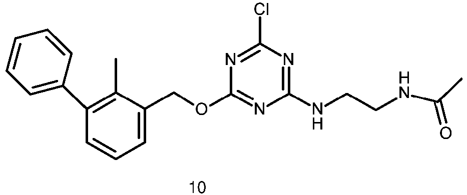

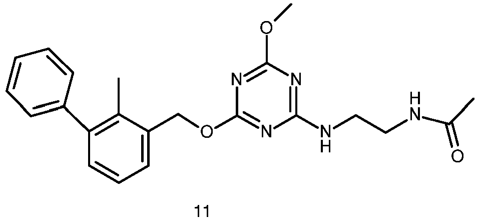

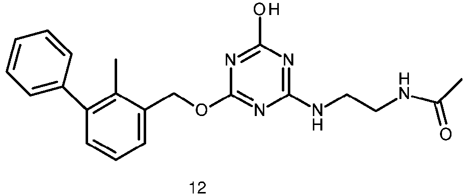

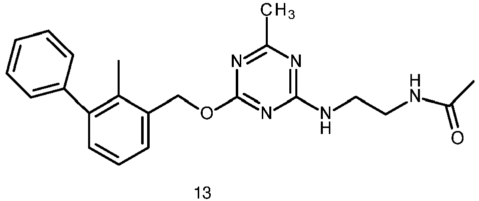

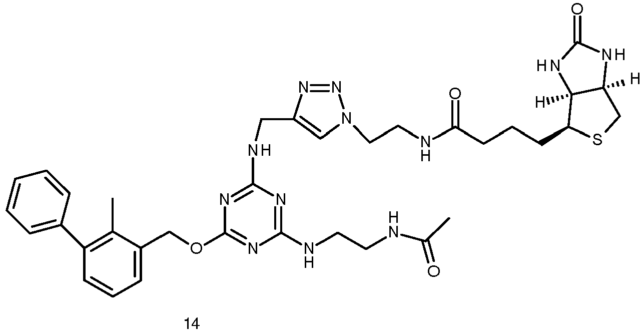

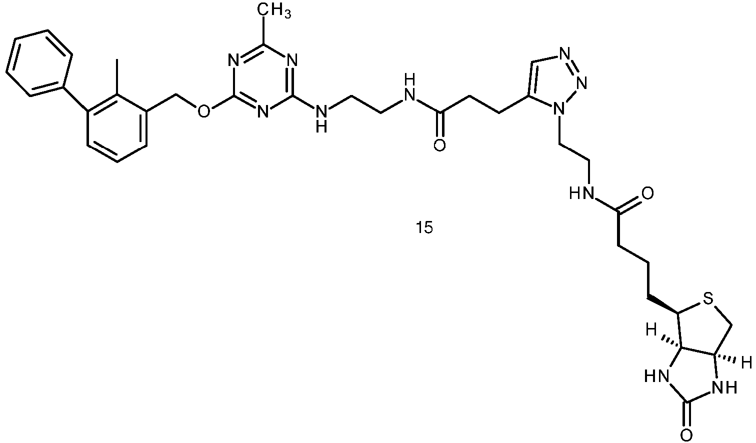

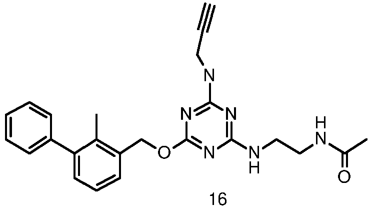

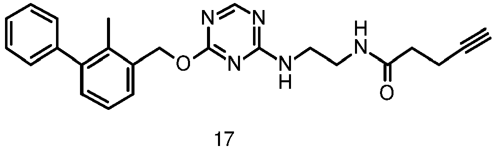

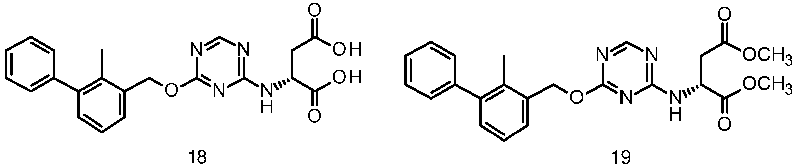

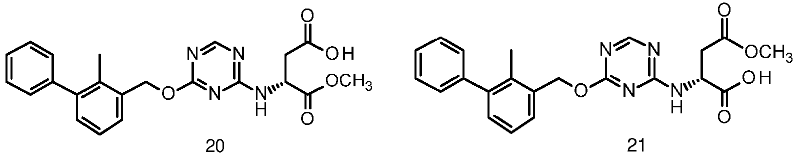

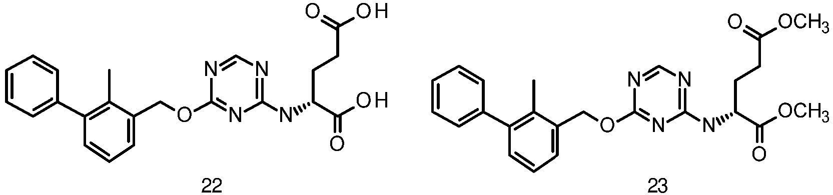

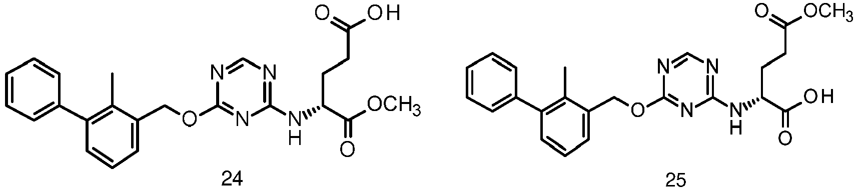

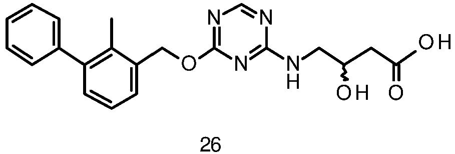

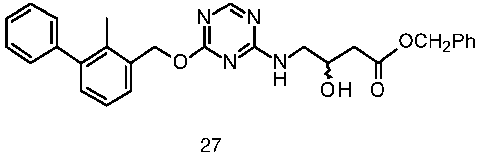

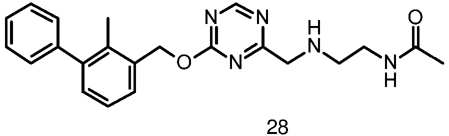

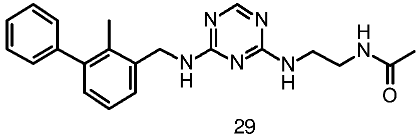

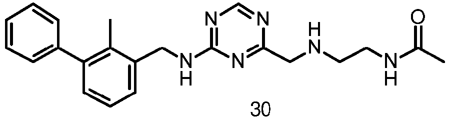

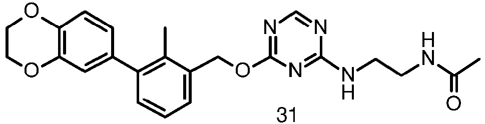

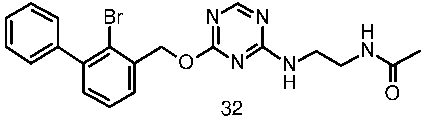

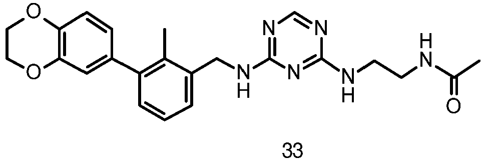

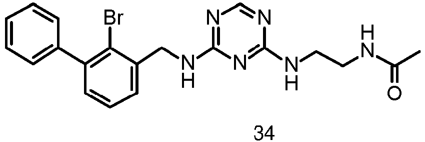

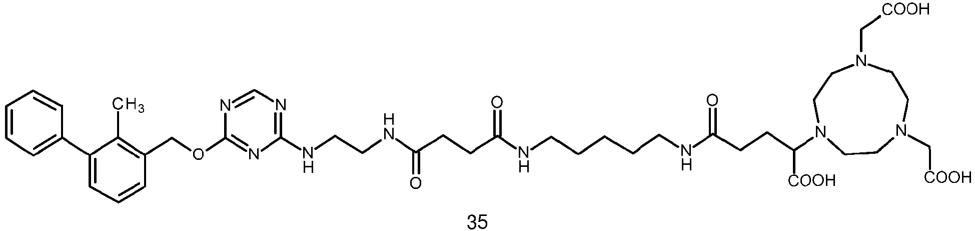

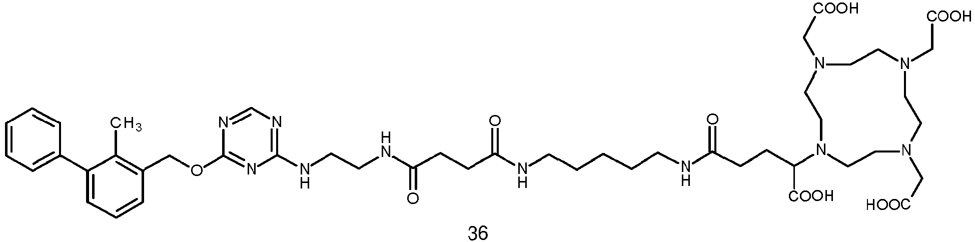

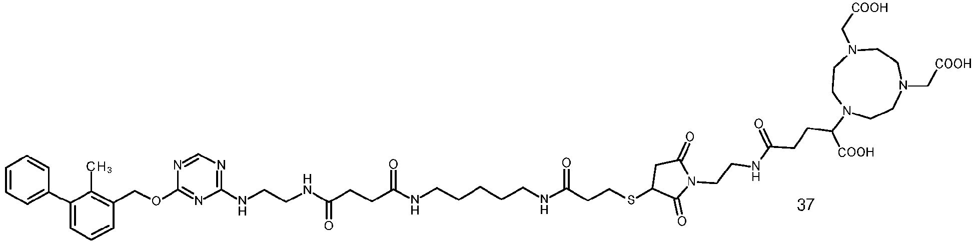

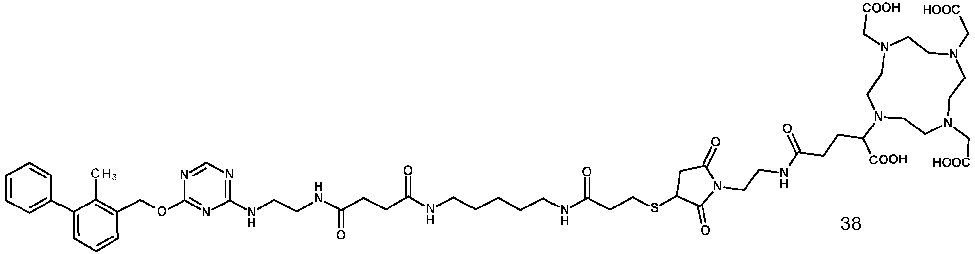

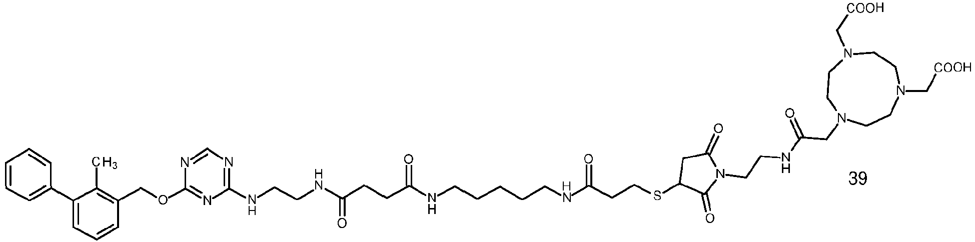

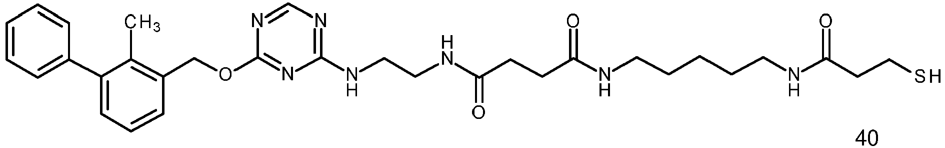

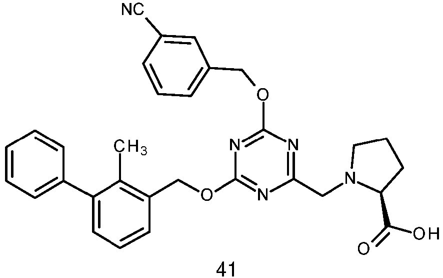

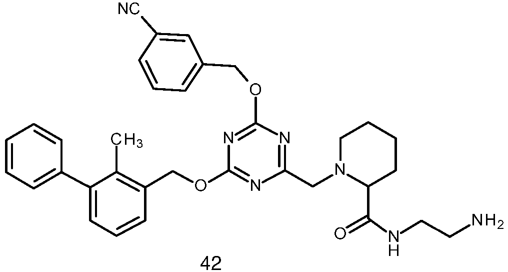

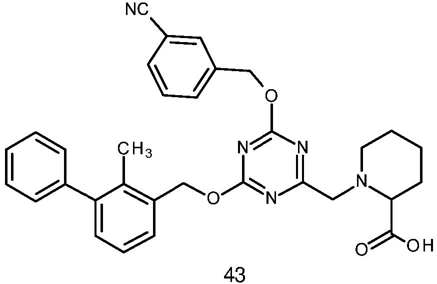

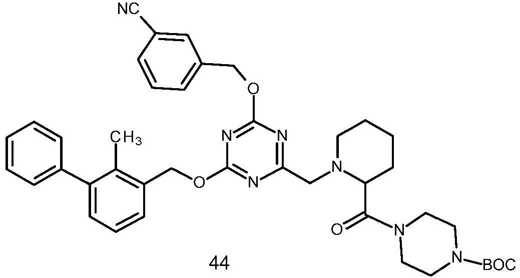

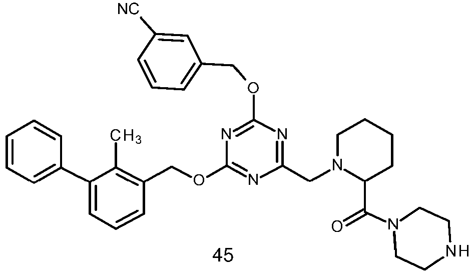

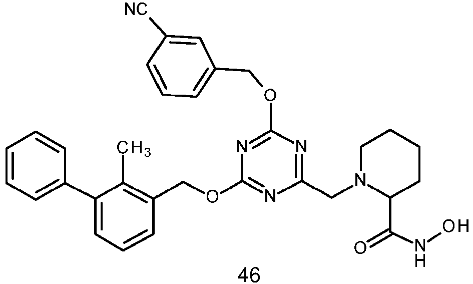

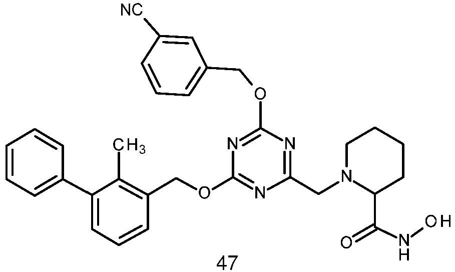

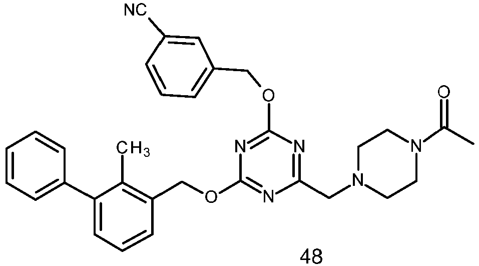

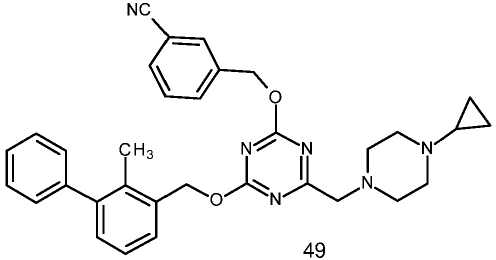

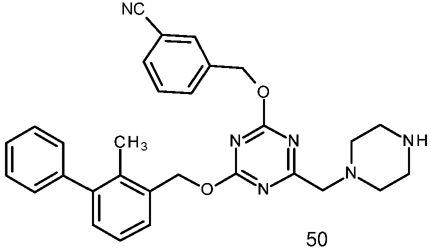

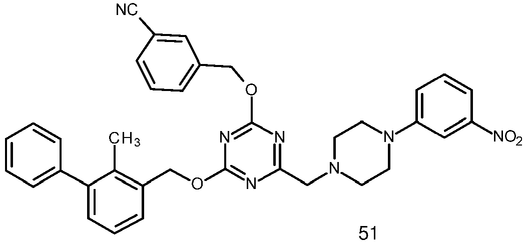

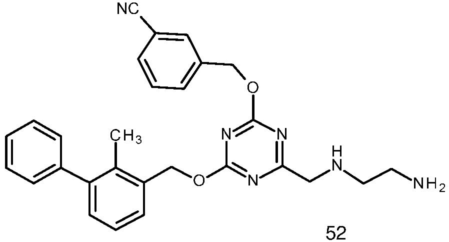

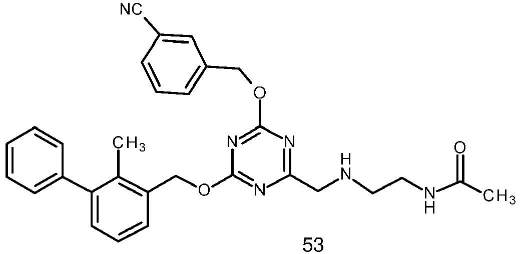

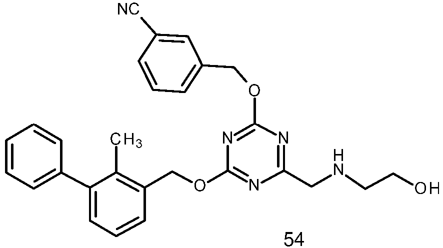

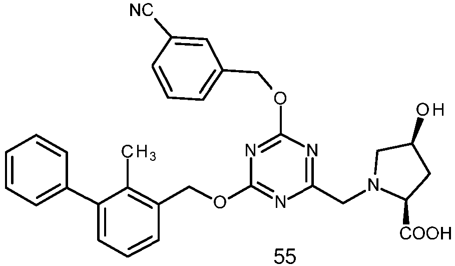

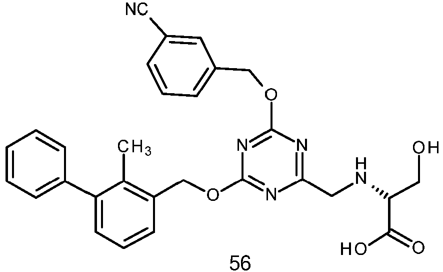

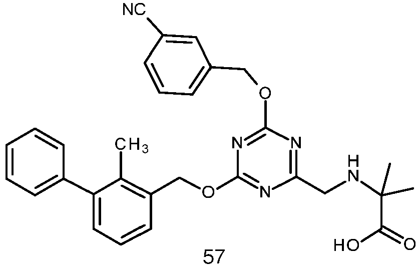

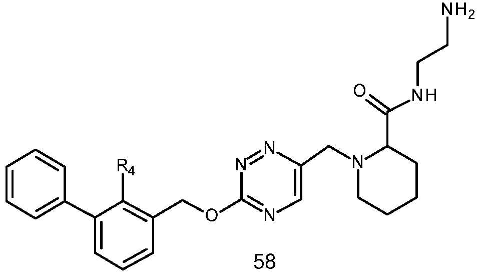

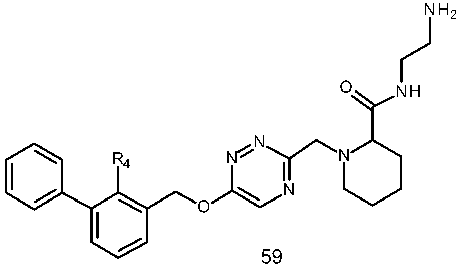

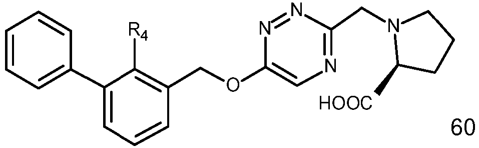

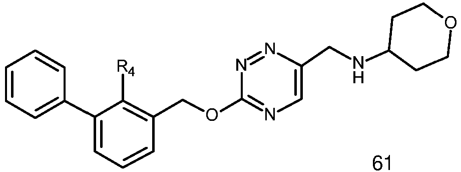

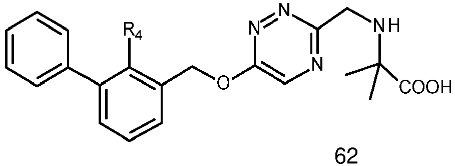

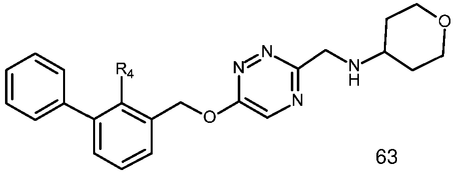

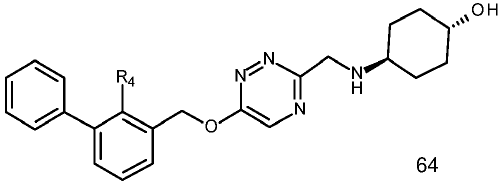

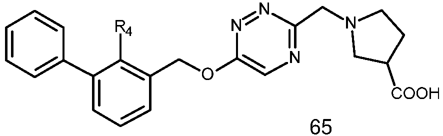

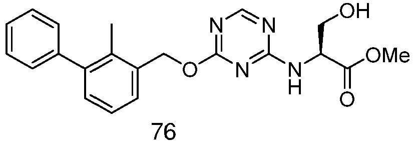

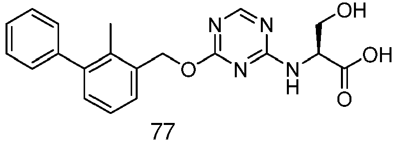

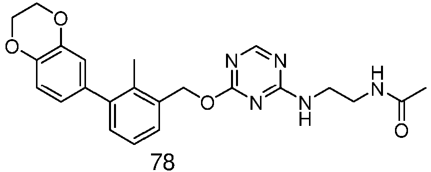

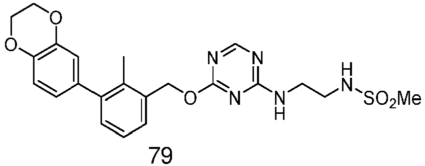

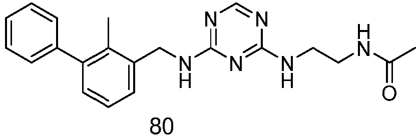

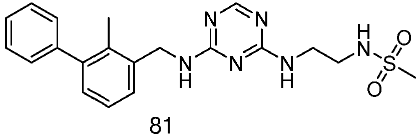

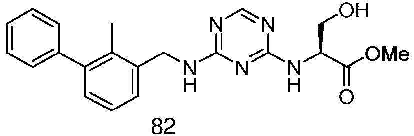

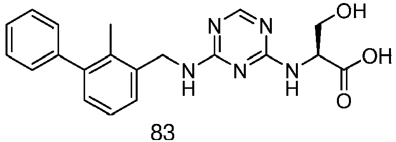

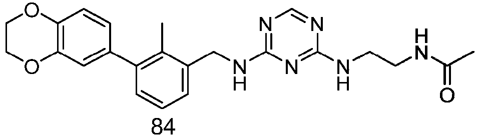

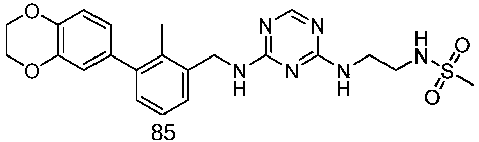

According to a further embodiment, the compounds of the present disclosure formula (III) (not part of the invention) 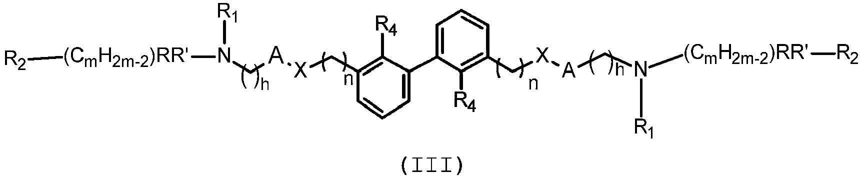 wherein:
**R₁** is selected from the group consisting of H and linear or branched C₁-C₄alkyl;
**R₂** is selected from the group consisting of
H,
C₁-C₄alkyl,
C₁-C₆cycloalkyl optionally substituted with OH,
C₁-C₆heterocycloalkyl,
phenyl optionally substituted with NO₂;
OH,
COOH,
COO-C₁-C₄alkyl,
COO-C₁-C₄alkylaryl,
CONH-C₁-C₆alkyl-NH₂,
CONHOH,
CO-C₁-C₆heterocycloalkyl,
NH₂,
NHSO₂-C₁-C₆alkyl,
SO₂NH₂,
SO₂NH-C₁-C₆alkyl,
NHCO-C₁-C₆alkyl optionally substituted with R₆, and 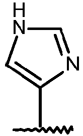
**m** is an integer between 0 and 4;
**R** and **R'** are selected independently from the group consisting of H, OH, C₁-C₄alkyl, COOH and COO-C₁-C₄alkyl;
**h** is an integer between 0 and 1;
**A** is a 1,3,5-triazine optionally substituted with R₃;
**X** is selected from the group consisting of O and NH;
**n** is an integer between 1 and 4;
**R₄** is selected from the group consisting of H, linear or branched C₁-C₄alkyl, halogen, CN, OH.

In particular, the compounds of the invention are selected from the group consisting of:

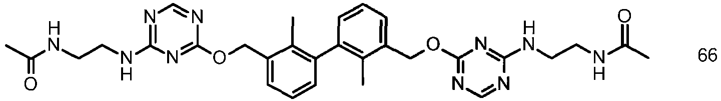

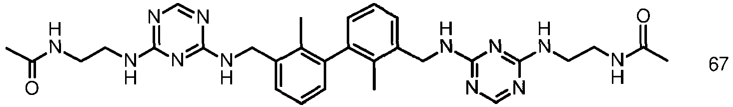

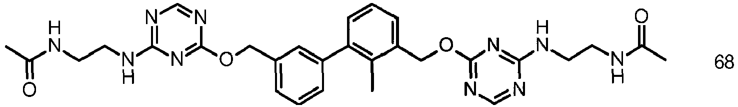

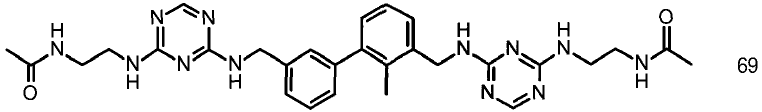

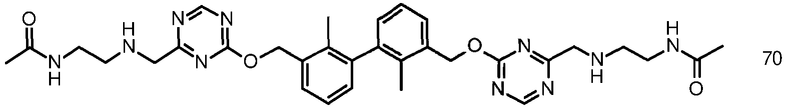

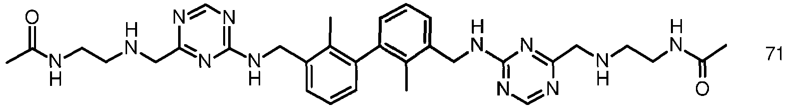

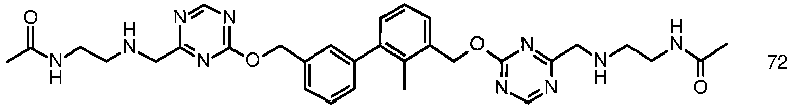

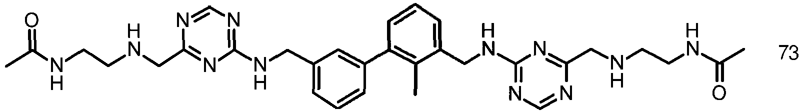

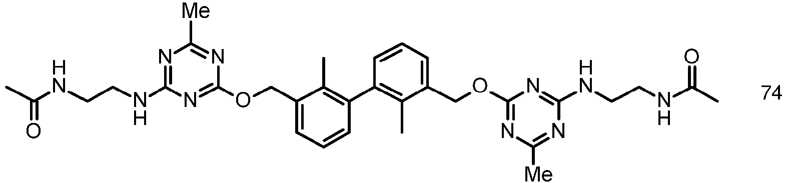

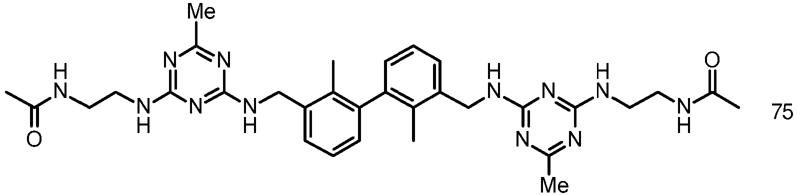

A second aspect of the present invention refers to a pharmaceutical composition comprising a compound of the present invention as shown above and at least one pharmacologically acceptable excipient.

A person skilled in the art is familiar with a whole variety of such excipient compounds suitable for formulating a pharmaceutical composition.

The compounds of the invention, together with a conventionally employed excipient may be placed in the form of pharmaceutical compositions and relative unit dosages, and in such form they may be used as solids, such as tablets or filled capsules, or liquids such as solutions, suspensions, emulsions, elixirs, or capsules filled with the same, all for oral use, or in the form of sterile injectable solutions for parenteral administration (including subcutaneous and intravenous use).

Such pharmaceutical compositions and relative unit dosage forms may comprise ingredients in conventional proportions, with or without additional active ingredients or compounds, and such unit dosage forms may contain any effective amount of the active ingredient commensurate with the expected daily dosage range to be used.

The pharmaceutical compositions containing a compound of this invention can be prepared in a manner known in the pharmaceutical art and comprise at least one active compound. Generally, the compounds of this invention are administered in a pharmaceutically effective quantity. The amount of the compound actually administered will typically be determined by a physician, in light of the relevant circumstances, including the disease to be treated, the route of administration chosen, the actual compound administered, the age, the weight, and the response of the individual patient, the severity of the patient's symptoms, and the like.

The pharmaceutical compositions of the present invention can be administered through a variety of routes, including oral, rectal, transdermal, subcutaneous, intravenous, intramuscular, intranasal and pulmonary routes. The compositions for oral administration may take the form of loose liquid solutions or suspensions, or loose powders. Most commonly, however, the compositions are presented in unit dosage forms to facilitate accurate dosing. The term "unit dosage forms" refers to physically discrete units suitable as unit dosages for humans and other mammals, each unit containing a predetermined amount of active material calculated to produce the desired therapeutic effect, in combination with a suitable pharmaceutical excipient. Typical unit dosage forms include ampoules or syringes of the pre-filled, pre-measured liquid compositions or pills, tablets, capsules or similar in the case of solid compositions.

Liquid forms suitable for oral administration may include a suitable aqueous or non-aqueous vehicle with buffers, suspending and dispersing agents, dyes, flavours and the like. Solid forms may include, for example, any of the following ingredients, or compounds of a similar nature: a binder such as microcrystalline cellulose, tragacanth gum or gelatin; an excipient such as starch or lactose; a disintegrating agent such as alginic acid, Primogel or maize starch; a lubricant such as magnesium stearate; a glidant such as colloidal silicon dioxide; a sweetening agent such as sucrose or saccharin; or a flavouring agent such as peppermint, methyl salicylate, or orange flavouring.

The injectable compositions are typically based on a saline solution or sterile injectable phosphate buffered saline solution or other injectable carriers known in the art.

The pharmaceutical compositions may be in the form of tablets, pills, capsules, solutions, suspensions, emulsions, powders, suppositories and as sustained release formulations.

If desired, the tablets may be coated using standard aqueous or non-aqueous techniques. In some embodiments, such compositions and preparations may contain at least 0.1 percent of active compound. The percentage of active compound in these compositions can certainly be varied, of course, and can be conveniently between about 1 percent and about 60 percent of the weight of the unit. The amount of active compound in such therapeutically useful compositions is such that the therapeutically active dosage will be obtained. The active compounds may also be administered intranasally as, for example, liquid droplets or sprays.

The tablets, the pills, the capsules, and the like may also contain a binder such as gum tragacanth, acacia, corn starch, or gelatin; excipients such as dicalcium phosphate; a disintegrating agent such as corn starch, potato starch, alginic acid; a lubricant such as magnesium stearate; and a sweetening agent such as sucrose, lactose, or saccharin. When a unit dosage form is a capsule, it may contain, in addition to the materials of the above type, a liquid carrier such as a fatty oil. Various other materials may be present as coatings or to modify the physical shape of the dosage unit. For example, the tablets may be coated with shellac, sugar, or both. A syrup or elixir may contain, in addition to the active ingredient, sucrose as a sweetening agent, methyl and propyl parabens as preservatives, a dye and a flavouring agent such as cherry or orange flavouring. To prevent decomposition during the transit through the upper portion of the gastrointestinal tract, the composition may be a coated enteric formulation.

Compositions for pulmonary administration include, but are not limited to, dry powder compositions consisting of the powder of a compound of the invention or a relative salt, and of the powder of a suitable carrier and/or lubricant. Compositions for pulmonary administration may be inhaled from any suitable dry powder inhaler device known to the person skilled in the art.

The administration of the compositions is performed under a protocol and at a dosage that is sufficient to reduce inflammation and pain in the subject. In some embodiments, in the pharmaceutical compositions of the present invention the active ingredient or the active ingredient(s) are generally formulated in dosage units. The dosage unit may contain 0.1 to 1000 mg of a compound of the present invention per dosage unit per daily administration.

In some embodiments, the effective amounts for a specific formulation will depend on the severity of the disease, disorder or condition prior to therapy, on the health status of the individual and on the response to the drug. In some embodiments the dose is in the range from 0.001% by weight to about 60% by weight of the formulation.

When used in combination with one or more of the other active ingredients, the compound of the present invention and the other active ingredient may be used in lower doses than when each is used individually.

Regarding formulations with respect to any variety of routes of administration, methods and formulations for drug administration are shown in Remington's Pharmaceutical Sciences, 17th edition, Gennaro et al. Eds., Mack Publishing Co., 1985, and Remington's Pharmaceutical Sciences, Gennaro AR ed. 20th edition, 2000, Williams & Wilkins PA, USA, and Remington: The Science and Practice of Pharmacy, 21th edition, Lippincott Williams & Wilkins Eds., 2005; and in Loyd V. Allen and Howard C. Ansel, Ansel's Pharmaceutical Dosage Forms and Drug Delivery Systems, 10th edition, Lippincott Williams & Wilkins Eds., 2014.

The components described above for orally administered or injectable compositions are purely representative.

The compounds of this invention can also be administered in sustained-release forms or by sustained-release drug delivery systems.

A third aspect of the present invention refers to a compound of the present invention as shown above for use as a medicament.

A compound of the present invention as shown above can be used in the prevention and/or treatment of a tumour, a viral infection, a bacterial infection, or a neurodegenerative disease.

In one embodiment, the tumour is selected from the group consisting of pancreatic cancer, bladder cancer, colorectal cancer, breast cancer, prostate cancer, renal cancer, hepatocellular cancer, lung cancer, ovarian cancer, cervical cancer, gastric cancer, oesophageal cancer, head and neck cancer, melanoma, neuroendocrine cancer, central nervous system cancer, brain cancer, bone cancer, soft tissue sarcoma, non-small cell lung cancer, small cell lung cancer or colon cancer, lymphocytic leukaemia, acute myeloid leukaemia, chronic lymphocytic leukaemia, small lymphocytic lymphoma, myelodysplastic syndrome, myeloproliferative disease, chronic myeloid leukaemia, multiple myeloma, non-Hodgkin's lymphoma, mantle cell lymphoma, follicular lymphoma, Waldestrom's macroglobulinemia, T-cell lymphoma, B-cell lymphoma or diffuse large B-cell lymphoma.

In one embodiment, the neurodegenerative disease is selected from the group consisting of schizophrenia, Alzheimer's disease, multiple sclerosis, Parkinson's disease, Huntington's chorea, spinocerebellar ataxia type 1, amyotrophic lateral sclerosis, Batten disease.

In one embodiment, the viral infection is caused by a virus selected from the group consisting of HIV, HBV, hepatitis A, B, C, D, herpes papillomavirus, influenza, COVID-19.

According to a further aspect of the invention there is further provided a combination therapy comprising a compound of the present invention as defined above and at least one additional anti-cancer agent selected from the group consisting of rituxan, doxorubicin, gemcitabine, nivolumab, pembrolizumab, atezolizumab, and nivolumab, pembrolizumab, atezolizumab, or ipilimumab or radiotherapy, and resection therapy, in particular comprising a checkpoint inhibitor of a monoclonal antibody or an antigen-binding moiety thereof and an additional therapeutic agent.

In addition, the compounds of the present invention can be used in molecular fluorescence tomography and positron emission tomography.

The utility of small molecules with IC50 for PD-L1 in the nanomolar range for positron emission tomography is documented in the literature (Bioorganic Chemistry, volume 115, October 2021, 105294; Bioorganic & Medicinal Chemistry Letters, volume 30, Issue 24, December 15, 2020, 127572) and, compared to monoclonal antibodies, and anti-PDL1 proteins currently in use, has the advantage of lower cost, better pharmacokinetics (radionuclide-labelled antibodies require in fact a biological half-life of several days to obtain high contrast in tissues), lower immunogenicity and greater penetration capacity in tissues.

Even in the case of the development of an electrochemical biosensor for the detection of soluble PD-L1, or present on the membrane of tumour cells or on the membrane of exosomes, there is a work in the literature that demonstrates that small molecules with a nanomolar IC50 towards PD-L1 can be used successfully for this purpose (Bioelectrochemistry, June 2021; 139:107742).

In light of this literature, it can be concluded that the compounds of the present invention are advantageously usable in molecular fluorescence tomography and positron emission tomography.

Further characteristics of the present invention will become apparent from the following description of some merely illustrative and non-limiting examples.

### Examples

### EXAMPLE 1.

### Synthesis of 2,4,6-trisubstituted cyanobenzyl triazines 1-3

The first phase in the synthesis of 2,4,6-trisubstituted cyanobenzyl triazines **1-3** involved the introduction of the biphenyl ether substituent on the triazine core (step a, Diagram 1). A nucleophilic substitution between (2-methyl-(1,1'-biphenyl]-3-yl)methanol and cyanuric chloride was performed in the presence of an organic base by slowly increasing the temperature from -20°C to room temperature, in order to minimize the risk of a double nucleophilic attack. The reaction proceeded rapidly, obtaining the desired dichlorobiphenyl ether triazine **INT-23** with good yields. The intermediate **INT-23** was subsequently reacted in a second substitution (step b) with (hydroxymethyl)benzonitrile, using equally mild reaction conditions but for a significantly longer reaction time. The desired chlorodiether triazine **INT-24** was obtained with good yields. Finally, a third substitution (step c) involved the use of a more reactive N-nucleophile (N-acetyl ethylenediamine - compound **1,** L-histidine methyl ester - compound **2**) under more stringent experimental conditions; both tri-substituted triazines **1 and 2** were obtained with good yields. Finally, standard basic hydrolysis of ester **2** led to the free carboxylate triazine **3** (step d, Diagram 1).

### Diagram 1

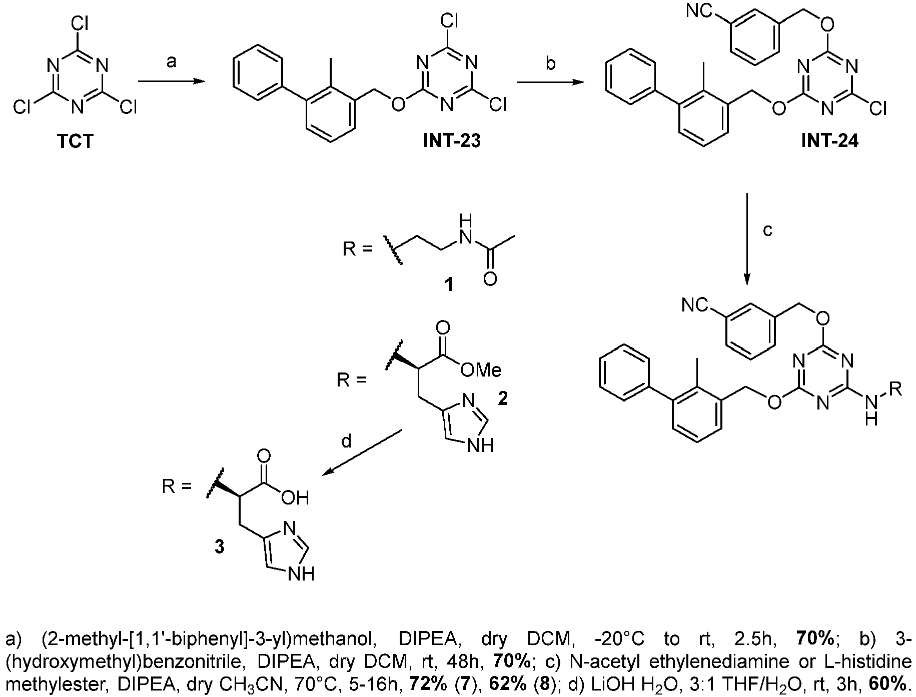

### EXAMPLE 2.

### Synthesis of 2,4-disubstituted triazines 4-6

The synthetic strategy envisaged for the preparation of 2,4-disubstituted triazines **4-6** (Diagram 2) is similar to that just described for the compounds **1-3** in Diagram 1.

The first nucleophilic substitution between (2-methyl-(1,1'-biphenyl]-3-yl)methanol and 2,4-dichlorotriazine was initiated at -20 °C, with gradual heating to room temperature (step a, Diagram 2). The desired chlorobiphenyl ether triazine **INT-25** was obtained with moderate yields. The introduction of N-acetyl ethylenediamine - **4** - or L-histidine methyl ester - **5** - in a second substitution (step b) was carried out with the same experimental protocol used for the tri-substituted triazines in Diagram 1 (compare step c, Diagram 1), obtaining both compounds with similar yields. Triazine **5** was then subjected to standard basic hydrolysis (step c, Diagram 2), producing the free carboxylate **6** with moderate overall yields.

### Diagram 2

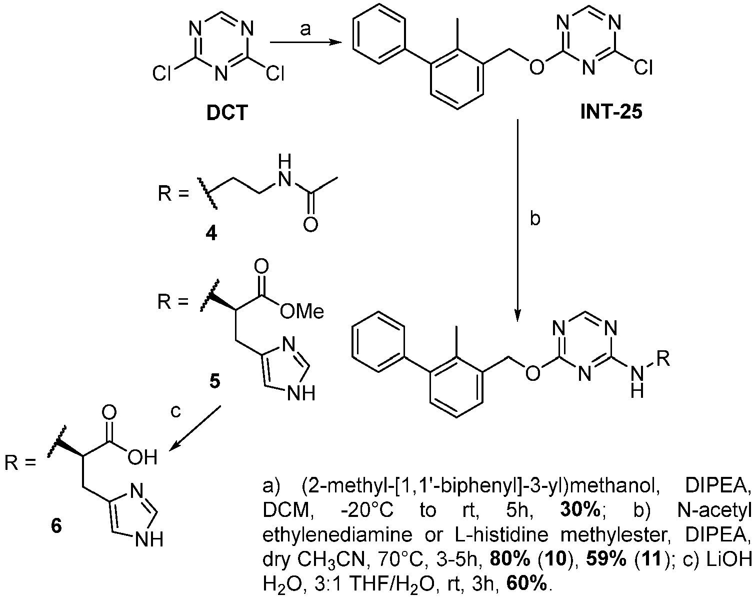

### EXAMPLE 3.

Polar chain modification: synthesis of 2,4-disubstituted triazines **7-9.**

The substitution of the terminal acetamide (compound **7)** with a direct sulphonamide (compound **8)** or a reverse methylsulphonamide (compound **9)** was explored. Their synthesis is shown in Diagram 3.

Starting from the biphenyl ether chlorotriazine **INT-25** described above, nucleophilic substitutions were carried out using N-(3-aminopropyl)acetamide **(7),** N-(2-aminoethyl)methanesulphonamide **(8)** or 2-aminoethanesulphonamide **(9)** under the same reaction conditions as reported above (step a, Diagram 3; compare with step b, Diagram 2). The 2,4-disubstituted triazines **7-9** were obtained with good to excellent yields.

### Diagram 3

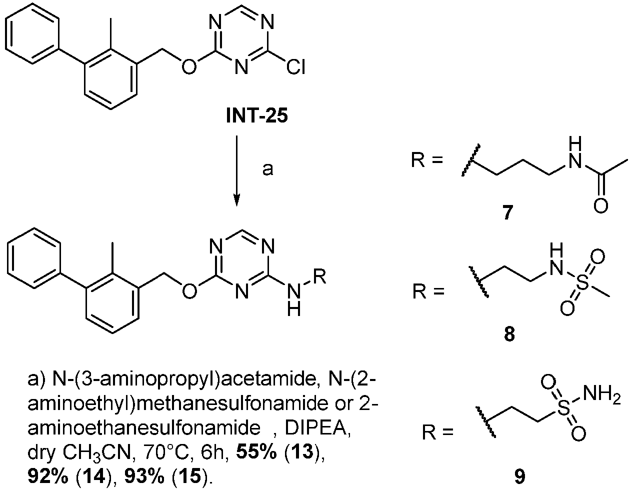

### EXAMPLE 4.

### Synthesis of 2, 4, 6-trisubstituted triazines 10-13

The 2-chloro trisubstituted compound **10** was obtained from the dichloro biphenyl ether **INT-23,** which underwent nucleophilic substitution (step b, Diagram 4) with N-(2-aminoethyl)acetamide. Once again, the reaction temperature was initially set at -20 °C and gradually increased to room temperature to avoid double substitution due to the higher strength of an N-nucleophile. The reaction proceeded as expected, yielding the 6-chloro substituted compound **10** with moderate non-optimised yields.

The compound **10** was then used to synthesise the hydroxysubstituted compound **11** using water as a nucleophile (step c). The reaction proceeded slowly, due to the low nucleophilicity of water, and the starting compound **10** could still be observed even after four days at 50 °C; the reaction was stopped to limit degradation of both the starting compound **10** and of the product **11,** which was isolated with a 30% yield.

Methoxy triazine **12,** was obtained by first introducing methanol (O-nucleophile) under the same mild conditions as before (step d). The reaction delivered dichloromethoxy triazine **INT-26** with good yields. A second nucleophilic substitution with (2-methyl-(1,1'-biphenyl]-3-yl)methanol was then performed under standard conditions, obtaining the chloro diether intermediate **INT-27** with good yields (step a). Finally, a third nucleophilic substitution was carried out with N-(2-aminoethyl)acetamide (step e, Diagram 4) under more stringent experimental conditions, obtaining the desired 4-methoxy triazine **12** with good yields.

### Diagram 4

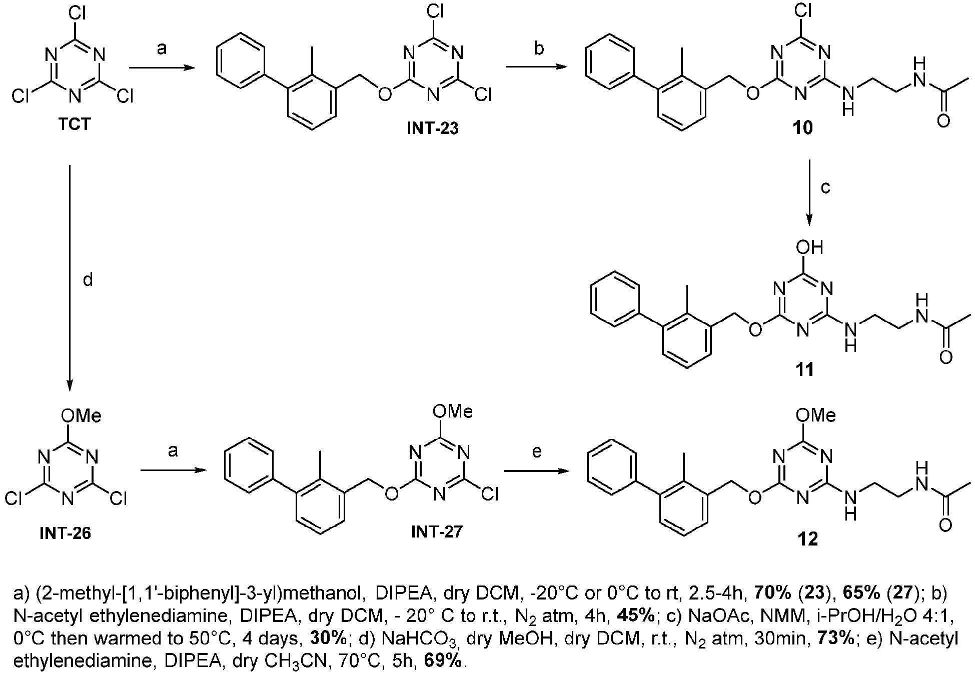

The last synthetic target, methyl triazine **13,** was prepared from the commercial dichloro-methyl triazine, as shown in diagram 5.

2,4-dichloro-6-methyl-1,3,5-triazine was subjected to a first nucleophilic substitution with 2-methyl-[1,1'-biphenyl]-3-yl)methanol (step a, Diagram 5). Assuming a lower reactivity of methyl triazine in nucleophilic substitutions, this reaction was performed at 40 °C, but led to multiple substitutions even before the starting material was consumed. Methyl chlorobiphenylalkoxy triazine **INT-28** was purified and isolated with non-optimised low yields. Finally, the intermediate **INT-28** was subjected to a second substitution with N-(2-aminoethyl)acetamide (step b, Diagram 5); more stringent reaction conditions were required to drive the reaction to completion and obtain the trisubstituted 6-methyl triazine **13** with moderate yields.

### Diagram 5

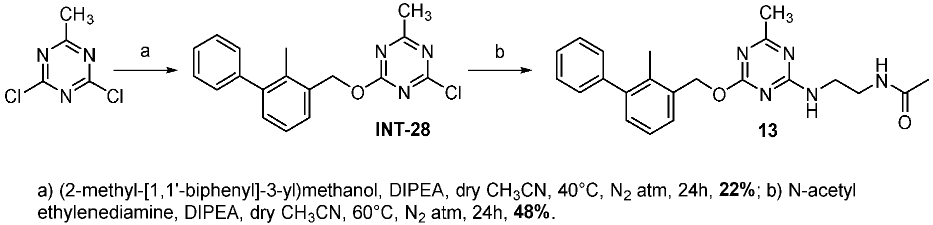

### EXAMPLE 5.

### Synthesis of biotinylated triazine 14

Dichlorobiphenyl ether triazine **INT-23** was reacted under mild nucleophilic conditions with propargylamine (step a, Diagram 6), obtaining chloroaminoalkynyl biphenyl ether triazine **INT-29** with good yields. The latter was reacted with N-(2-aminoethyl)-acetamide (step b) under stronger conditions, obtaining the alkynyl triazine **INT-30** with good yields. Finally, a 1,3-dipolar Huisgen copper-catalysed cycloaddition between alkenyl triazine **INT-30** and azido biotinamide **INT-31** under standard conditions (step c) yielded the desired derivative **14** with moderate yields. Azido biotinamide **INT-31** was prepared by simple amidation of biotin with commercial 2-azidoethylamine (step d, Diagram 6) with good yields.

### Diagram 6

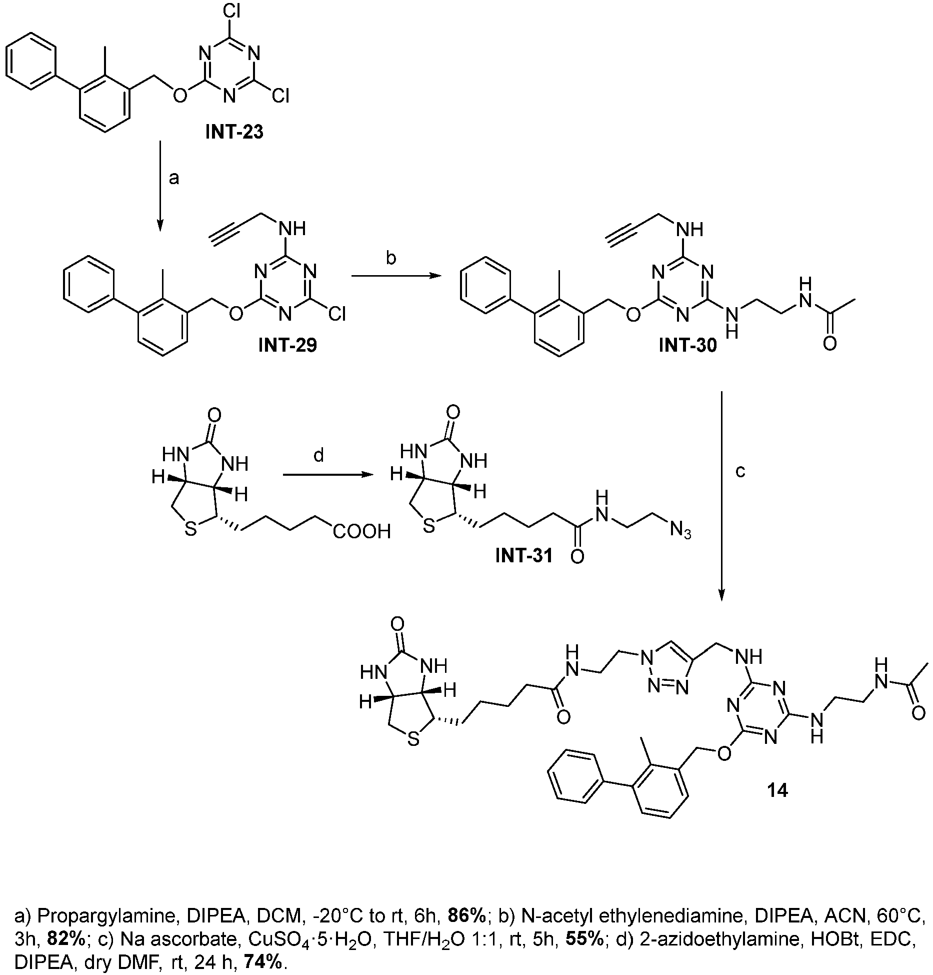

### EXAMPLE 6.

### Synthesis of biotinylated triazine 15

The previously described chlorobiphenyl ether **INT-25** underwent nucleophilic substitution with N-(2-aminoethyl)pent-4-ynamide **INT-32** (step a, Diagram 7), obtaining alkynyl triazine **INT-33** with moderate yields. Biotinylated disubstituted triazine **15** was obtained with moderate yields via a 1,3-dipolar Huisgen copper-catalysed cycloaddition between the intermediate **INT-33** and the azido biotinamide **INT-31** under standard conditions.

### Diagram 7

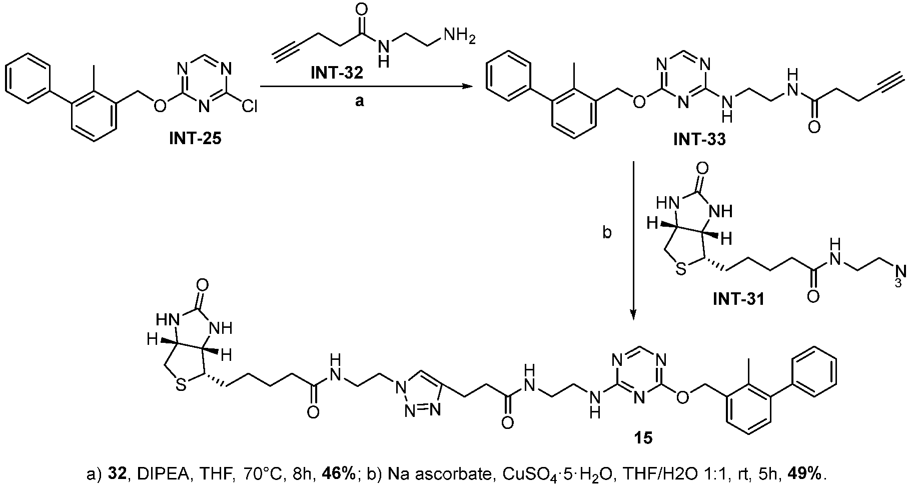

### EXAMPLE 7.

Polar chain modification: synthesis of 2,4-disubstituted triazine **77.**

The substitution of the terminal acetamide (compound **7)** with a serine (compound **77)** was further explored. The synthesis, described in Diagram 8, starts from the previously described chlorobiphenyl ether **INT-25,** which is subjected to nucleophilic substitution using L-serine methyl ester hydrochloride under the same reaction conditions as reported above (step a, Diagram 8; compare with step b, Diagram 2), obtaining the 2,4-disubstituted triazine **76** with good yield. Finally, the methyl ester is removed by standard basic hydrolysis (step b, Diagram 8), producing the free carboxylate **77.**

### Diagram 8

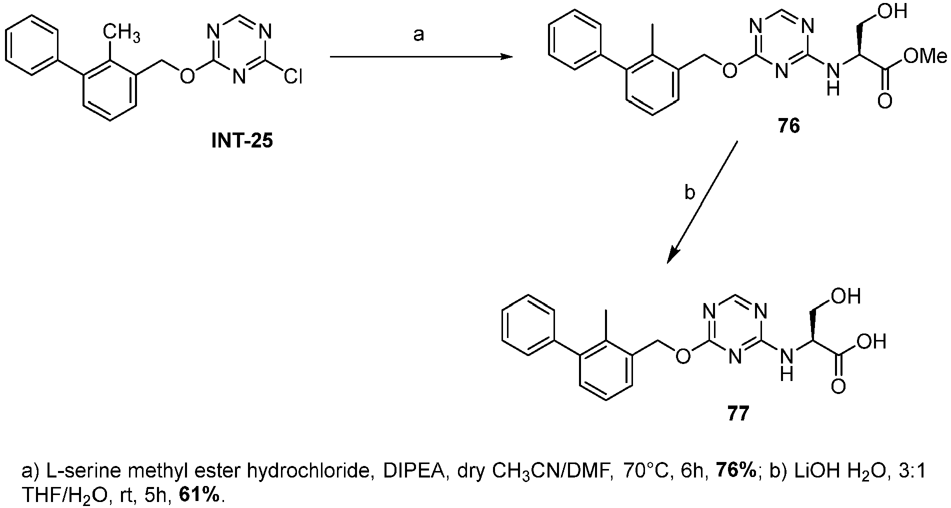

### EXAMPLE 8.

Biphenyl modification: synthesis of 2,4-O,N-disubstituted triazines **78** and **79.**

The synthesis strategy for the preparation of **78** and **79** involved, as a first reaction step, the attack of the polar chain at low temperature (step a, Diagram 9), which led the intermediates functionalised with N-acetyl ethylenediamine **(INT-34)** and N-(2-aminoethyl)methanesulphonamide**(INT-35)**, respectively, to be obtained with acceptable yields. A second nucleophilic reaction under more drastic conditions, using an inorganic base (K₂CO₃) and by increasing the temperature (up to 100 °C) in the presence of (3-(2,3-dihydrobenzo[b][1,4]dioxin-6-yl)-2-methylphenyl)methanol, synthesised as reported in the literature (Guzik et al., J. Am. Chem. Soc. 2017, 60, 5857-5867), resulted in the desired products **78** and **79** being obtained with low yields.

### Diagram 9

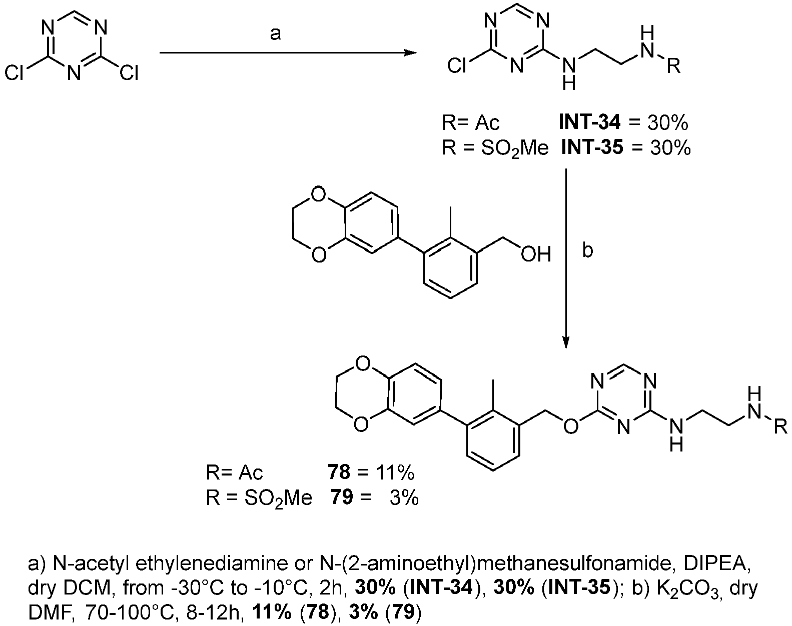

### EXAMPLE 9.

Modification of the connection between triazine and biphenyl: synthesis of the 2,4-N,N-disubstituted triazines **80, 81, 82, 83, 84** and **85.**

The synthesis of these derivatives involves an initial nucleophilic substitution under mild conditions (step a, Diagram 10) between 2,4-dichlorotriazine and the biphenyl derivatives functionalised with the amine group (2-methyl-[1,1'-biphenyl]-3-yl)methanamine or (3-(2,3-dihydrobenzo[b][1,4]dioxyn-6-yl)-2-methylphenyl)methanamine, prepared from the respective alcohols using optimised literature procedures (Guo et al., J. Med. Chem. 2020, 63, 13825-13850). The desired chlorotriazines **(INT-37** and **INT-38)** are obtained with moderate yields. A second nucleophilic substitution step delivers the desired products with good yields using N-acetyl ethylenediamine as nucleophile **(80** starting from **INT-37, 84** starting from **INT-38),** N-(2-aminoethyl)methanesulphonamide(**81** starting from **INT-37, 85** starting from **INT-38)** or L-serine methyl ester **(82** starting from **INT-37)** under the same reaction conditions as reported above (step b, Diagram 10; compare with step b, Diagram 2). Finally, the methyl ester in **82** was removed by basic hydrolysis under standard conditions delivering the carboxylate **83** with moderate yields.

### Diagram 10

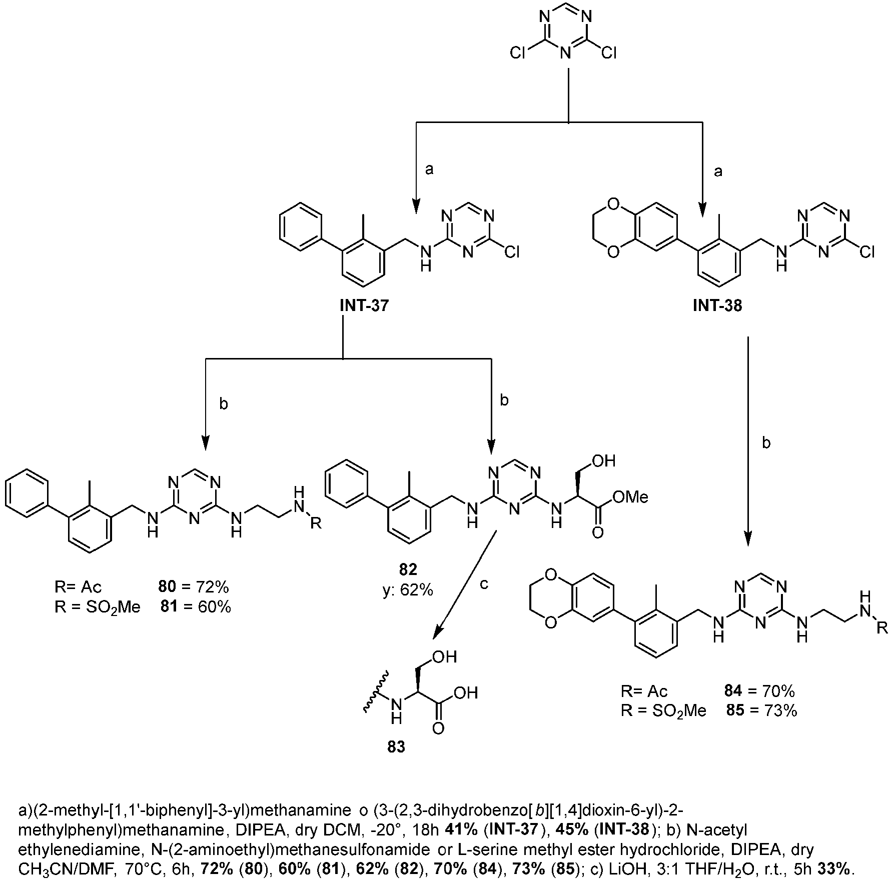

### EXAMPLE 10.

Pseudo-dimeric ligands: synthesis of bis-triazine biphenyls **66, 67, 68,** and **69.**

Pseudo-dimeric ligands centred on double substitution around the biphenyl core were designed and synthesised using a strategy shown in the two diagrams below. As a first step, it was necessary to synthesize a biphenyl core bearing the double hydroxyl (mono- **INT-39** and dimethyl **INT-40** compounds, obtained with good yields via Suzuki reaction with boronic acid or ester, steps a or b, Diagram 11) and amine functionalisation (mono- **INT-43** and dimethyl **INT-44** compounds, obtained with moderate yields from **INT-39 and INT-40** respectively by nucleophilic substitution with azide via bromide to give mono- **INT-41** and dimethyl-azido **INT-42** derivatives, steps c or b, Diagram 11; and subsequent catalytic hydrogenation to give mono- **INT-43** and dimethyl **INT-44** bis-amines, step e, Diagram 11).

### Diagram 11

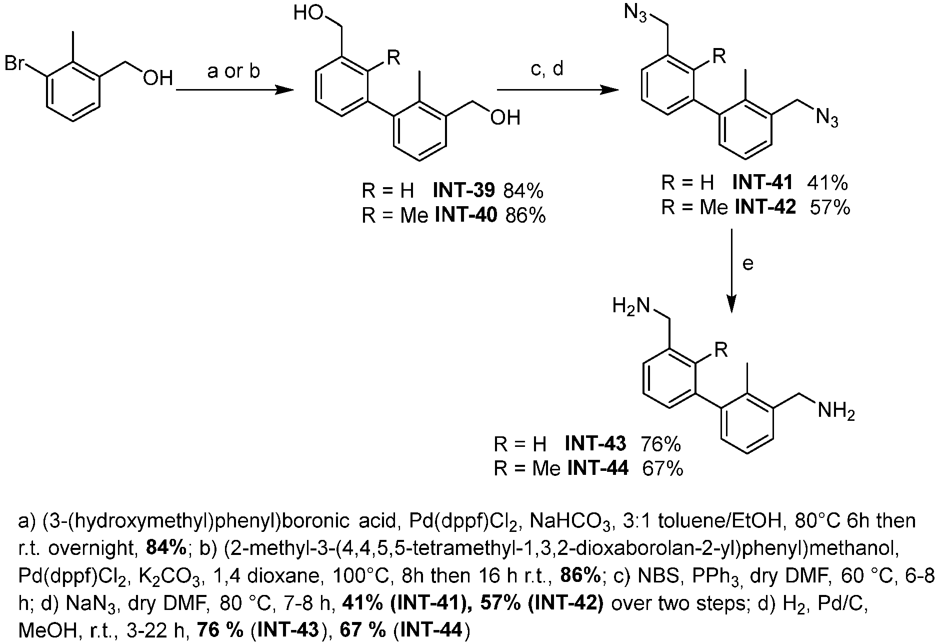

The bis-amines **INT-43** and **INT-44** were then reacted with the mono-substituted triazine **INT-34** in a nucleophilic substitution (step a, Diagram 12), delivering the respective bis-triazine biphenyl pseudo-dimers connected through amine bond **67** and **69** with modest yields.

More drastic nucleophilic substitution conditions were necessary to obtain the conjugated dimers through ether bonding (step b, Diagram 12). Also in this case, starting from the amino alcohols **INT-39** and **INT-40** respectively, the bis-triazine biphenyl pseudo-dimers connected through ether bond **66** and **68** are isolated with low yields.

### Diagram 12

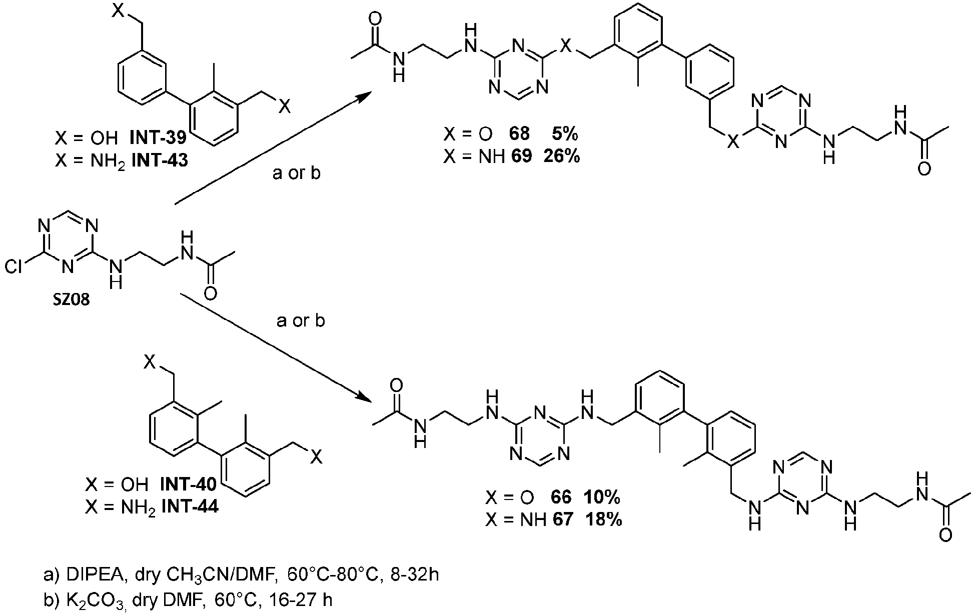

### EXAMPLE 11.

### NMR binding assay

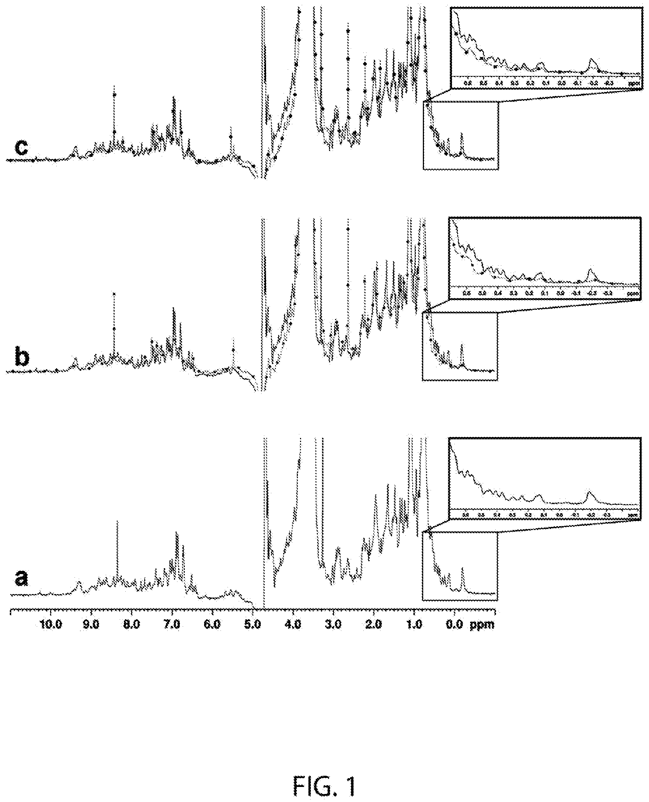

Macromolecule-based 1D ¹H NMR experiments were used to detect the interaction between the PD-L1 protein, expressed and purified as previously described, and the compounds of the invention. The 1D ¹H NMR spectra of 10 M PD-L1 were acquired in the presence of different ligands (protein:ligand ratio equal to 1:1 and 1:10), and the proton NMR linewidth of the protein signals were analysed to discover a new ligand. In particular, in these experiments, the chemical shift as well as the decreases in the intensities of PD-L1 resonance signals were monitored to follow the formation of the ligand-protein complex (Figure 1).

In the present NMR assay, we compared the 1D ¹H NMR spectra of PD-L1 in the presence of the compounds of the invention with those of free PD-L1 and PD-L1 in the presence of the ligand BMS-202, which was used as a reference control. By way of example, Figure 1 shows the comparison between the 1D ¹H NMR spectra of the free protein (Figure 1 a), the protein in the presence of BMS-202 (Figure 1 b), and the protein in the presence of compound 4 (Figure 1 c). When the compound BMS-202 is added to PDL-1, even in stoichiometric ratio, a decrease in the intensity of the free protein signals, as well as the appearance of new signals are observed (Figure 1 a). Comparable results were obtained with compound 4. In fact, the 1D ¹H-NMR spectrum of PD-L1 in the presence of compound 4 is similar to that of PD-L1 induced by the presence of BMS-202: the signal line broadening of the two protein-ligand complexes is similar and much greater than that of the free protein (Figure 1 a). This unequivocally confirms the formation of the compound 4-PD-L1 complex, presumably very similar to that of the BMS-202-PD-L1 complex since the spectra of the two complexes are very similar. The formation of the complex between PD-L1 and the aforementioned compounds is best evidenced by comparing the aliphatic regions of the protein spectra in the absence (Figure 1 a) and in the presence of BMS-202 (Figure 1 b) and compound 4 (Figure 1 c).

Noteworthy, comparing the NMR spectra (Figure 1) with the IC₅₀ values measured with the HTRF assay (Table 1), it is clear that the macromolecule-based experiment 1D ¹H NMR was effective in identifying new PD-L1 ligands, although a quantitative assessment of binding potency cannot be measured with this methodology.

### EXAMPLE 12.

### HTRF assay

As further confirmation of the results of the NMR assay and to classify the compounds of the invention based on their in vitro ability to inhibit PD-1/PD-L1 interaction, a homogeneous fluorescence binding assay resolved over time (HTRF) was used. This assay allows a simple and rapid characterisation of the inhibitors in a high-throughput format. Basically, it uses labelled human recombinant immune checkpoint partners (hPD1 and hPD-L1) and labelled anti-tag reagents for HTRF detection. In more detail, the interaction between hPD-L1 (Tag 1) and hPD1 (Tag2) is detected using Europium-labelled anti-Tag1 (HTRF donor) and XL665-labelled anti-Tag2 (HTRF acceptor) . After hPD-L1 binds hPD1, the donor and acceptor antibodies are in close proximity, so excitation of the donor antibody triggers fluorescence resonance energy transfer (FRET) towards the acceptor antibody, which in turn specifically emits at 665 nm. This signal is directly proportional to the extent of the hPD1/hPD-L1 interaction. Thus, the compounds capable of inhibiting the PD1/PD-L1 interaction induce a reduction in HTRF signal.

As shown in Table 1, among the compounds of the invention, the compound **4** showed the highest inhibitory potency with an IC₅₀ value of 115 (± 24) nM. Therefore, this compound was selected for subsequent biophysical and biological evaluations. The structures represented in Table 1 differ in the R₂ and/or R₃ substituents, highlighting a clear structure-activity relationship, which in turn helps to better characterise the requirements for the compounds.

**Table 1**

| | | | | |
|---|---|---|---|---|
| General Structure | 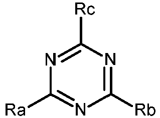 | | | |

| Comp. | Ra | Rb | Rc | IC50 (µM) |
|---|---|---|---|---|
| 1 (RS30) | 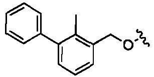 | 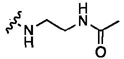 | 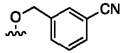 | > 5 |
| 2 (RS47) | 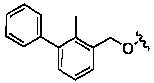 | 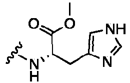 | 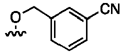 | > 5 |
| 3 (RS34) | 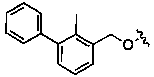 | 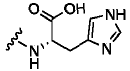 | 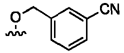 | > 5 |
| 4 (RS39) | 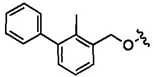 | 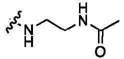 | 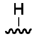 | 0.115 ± 0.02 4 |
| 5 (RS46) | 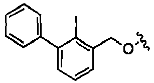 | 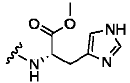 | 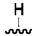 | 4.196 ± 0.680 |
| 6 (RS40) | 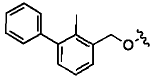 | 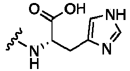 | 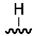 | > 5 |
| 7 (MAP55) | 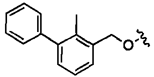 | 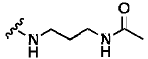 | 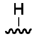 | 2.145 ± 0.51 9 |
| 8 (MAP 61) | 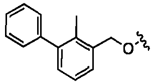 | 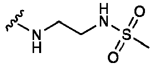 | 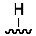 | 0.241 ± 0.058 |
| 9 (MAP62) | 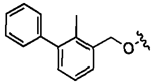 | 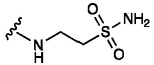 | 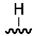 | 0.551 ± 0.133 |
| 10 (PO02) | 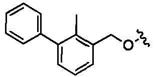 | 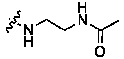 | 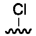 | 0.315 ± 0.016 |
| 11 (PO05) | 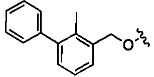 | 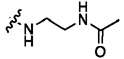 | 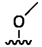 | 1.532 ± 0.075 |
| 12 (PO06) | 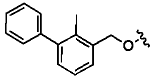 | 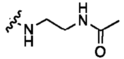 | 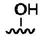 | > 5 |
| 13 (PO08) | 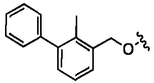 | 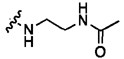 | 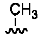 | > 5 |
| 14 (GA211) | 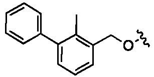 | 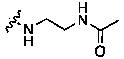 | 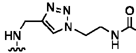 | n.a.^{a} but active in the cell |
| 15 (GA212) | 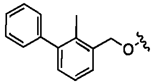 | 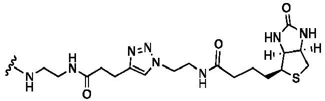 | 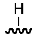 | n.a.^{a} but active in the cell |
| 26 (MF15) | 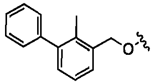 | 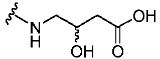 | 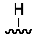 | > 10 |
| 100^{b} (RS13) | 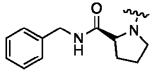 | 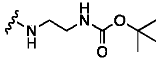 | 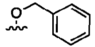 | > 5 |
| BMS202 | | | | 0.022 ±0.003 |

| | | | | |
|---|---|---|---|---|
| ^{a} IC₅₀ determination was not possible due to the interference of biotin with the assay. ^{b} compound 100 was synthesised as a negative control. | | | | |

**Table 2**

| Comp. | Structure | IC50 |
|---|---|---|
| 76 (MF55) | 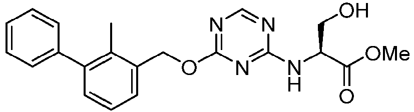 | 692 nM |
| 77 (MF57) | 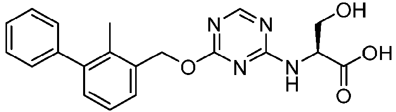 | > 10 µM |
| 80 (MF48) | 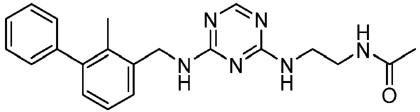 | > 10 µM |
| 81 (GA329) | 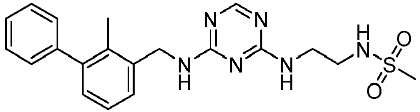 | > 10 µM |
| 82 (GA334) | 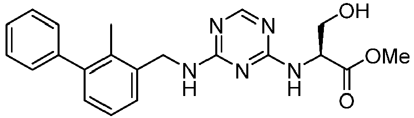 | > 10 µM |
| 83 (GA339) | 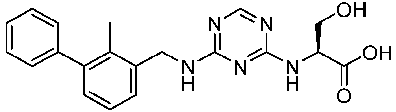 | > 10 µM |
| 84 (GA335) | 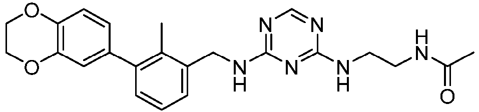 | > 10 µM |
| 85 (GA341) | 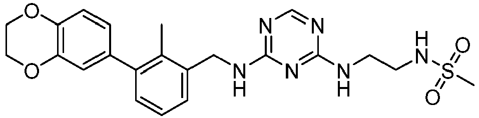 | > 10 µM |

### EXAMPLE 13.

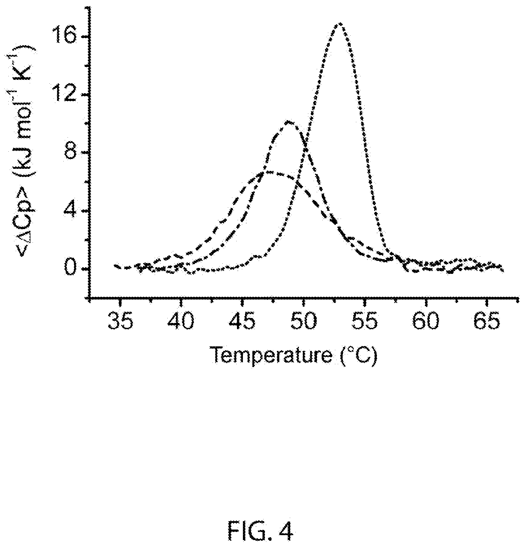

Differential scanning calorimetry (DSC) experiments were conducted to compare the behaviour of triazine 4 with that of compound BMS202 in binding and stabilising the PD-L1 protein. It is known that if a compound binds preferentially to a folded protein, the melting temperature (Tm) of the latter generally increases, and the tighter the bond is, the higher the Tm. Thus, DSC experiments in which the PD-L1 protein (32 µM) was heated in the absence and in the presence of both ligands (32 µM), to determine changes in Tm were performed. When no ligand was present, a Tm value, corresponding to the maximum of the respective thermogram peak, of 46.5 (±0.5) °C was observed. In the presence of compound 10 or BMS202, Tm values of 49.0 (±0.5) °C and 53.0 (±0.5) °C were observed, respectively. Thus, DSC analysis showed that both compounds significantly shifted the PD-L1 fusion peak, indicating for both a direct binding with a change in the Tm of the protein (ΔTm) of 2.5 and 6.5 °C for compound 10 and BMS202, respectively (see Figure 4).

### EXAMPLE 14.

### Molecular modelling

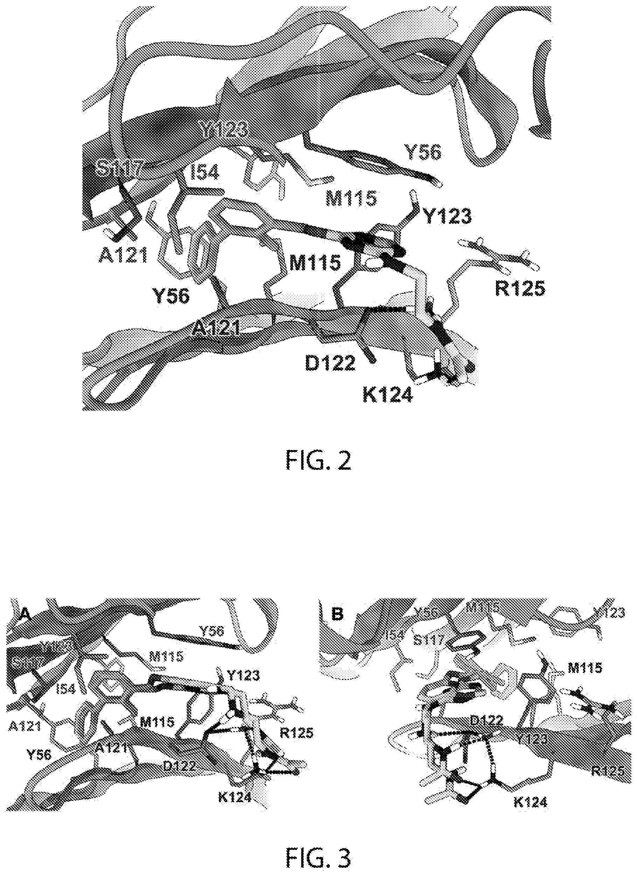

In order to clarify at the atomistic level the binding mode of compound **4** to the PD-L1 receptor, molecular docking studies were performed. Like for the selection of the three-dimensional structure of the protein, the X-ray complex of the PD-L1 homodimer (monomers A and B) was chosen with the known BMS202 inhibitor (PDB code: 5J89), based on the structural similarity between this compound and the compounds of the invention. The docking of compound **4** provided that this molecule can be hosted, in a similar manner to BMS202, in the so-called cylindrical hydrophobic pocket defined at the interface between the two PD-L1 monomers. In detail, the 2-methylbiaryl function sinks to the lower part of the binding site, involving a T-shaped stacking interaction with the phenolic function of _{A}Y56 as well as multiple lipophilic contacts with the side chains of _{A}M115, _{B}M115 and _{B}A121 (Figure 2) similar to what is found for BMS202. Furthermore, while the triazine core establishes a π-stacking with the side chain of _{B}Y56, the N-(2-aminoethyl)acetamide chain interacts via a bridge with the side chains of _{A}K124 and _{A}D122, and hydrogen bonds with the cationic head of _{A}K124. Although the binding mode envisaged for compound **4** is mostly superimposable on the crystallographic position of BMS202 (Figure 3), important differences are observed in the positioning of the central nuclei and in the interaction between the polar side chains and the amino acids of the receptor. The aforementioned differences are mainly due to the fact that the triazine core, unlike pyridine, supports meta- and not para-substitution, so it rearranges spatially towards Y123 to correctly orient the biphenyl function and the polar side chain together with the cylindrical pocket (Figure 3). Along the same lines, calculations suggest that the substitution of the N-(2-aminoethyl)-acetamide chain in compound **4** with bulky amino acids (such as histidine in compounds **5** and **6)** poses problems of simultaneous optimal arrangement of the biaryl function and of the polar side chain. Consequently, small changes in the hydrophilic alkyl amino chain of the ligand (e.g. compounds **7** and **8)** do not significantly affect the overall ligand-receptor recognition process.

## Claims

1. Compound selected from the group consisting of:

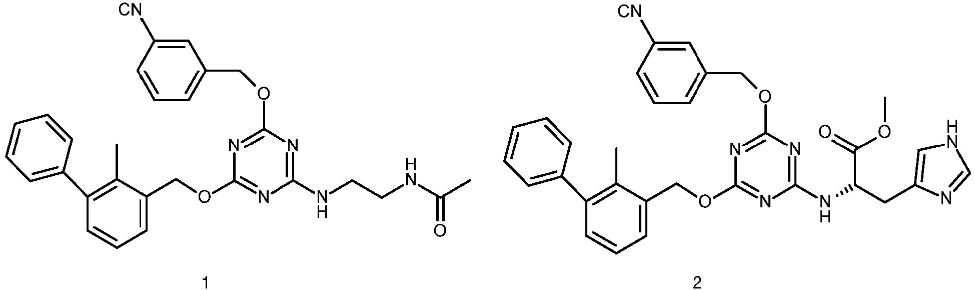

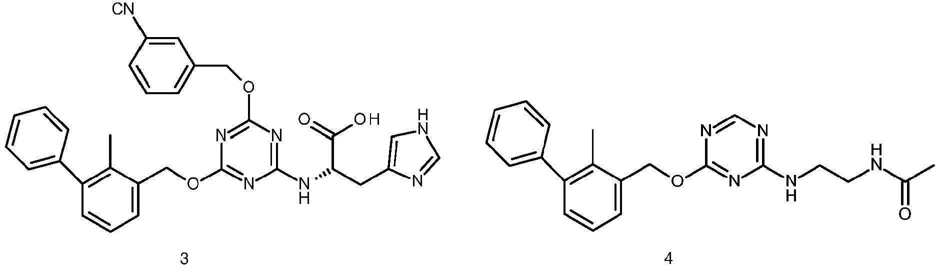

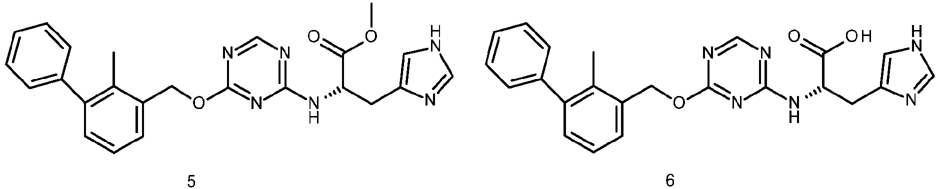

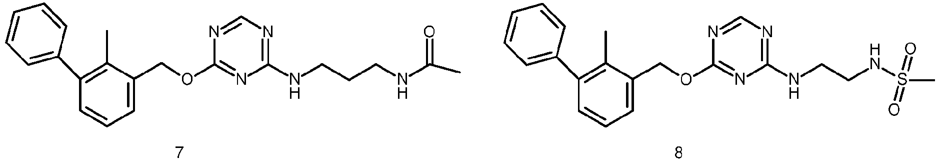

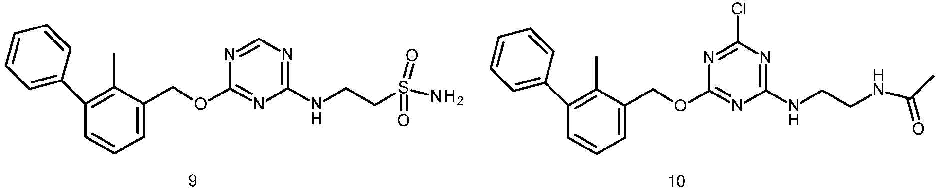

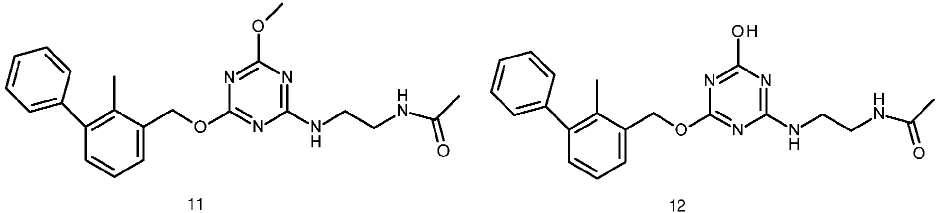

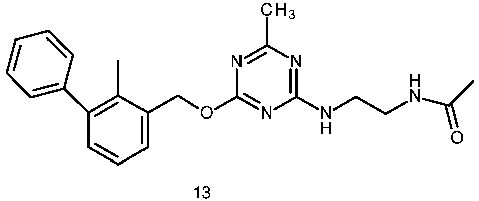

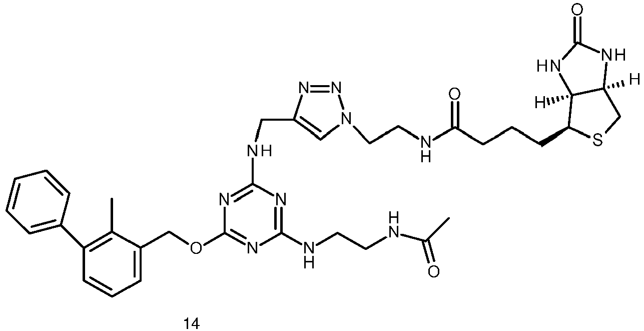

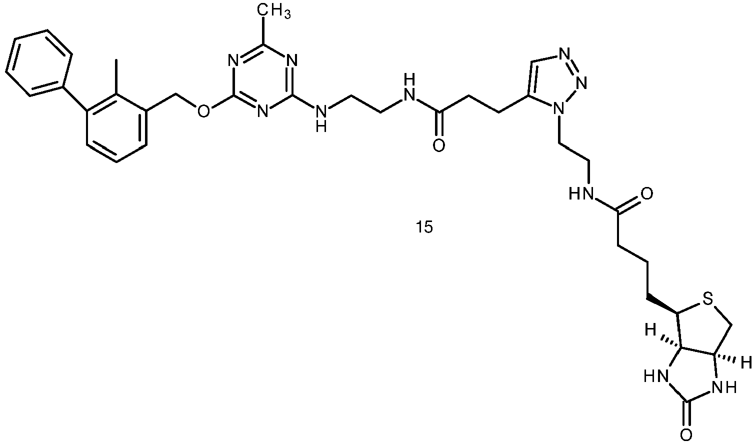

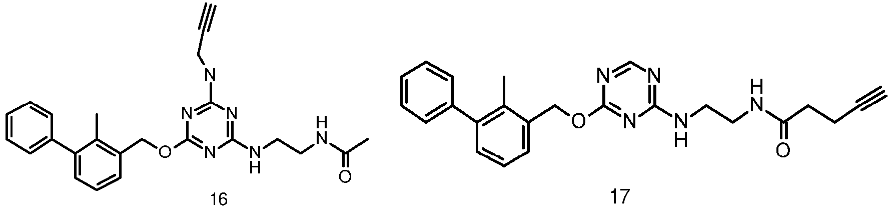

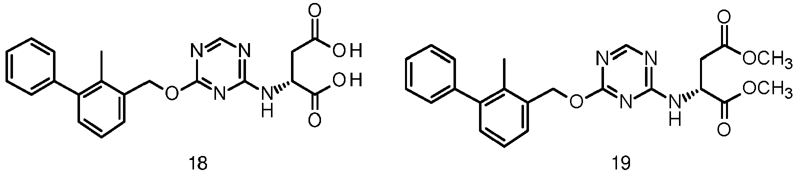

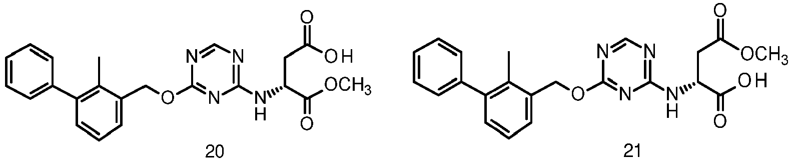

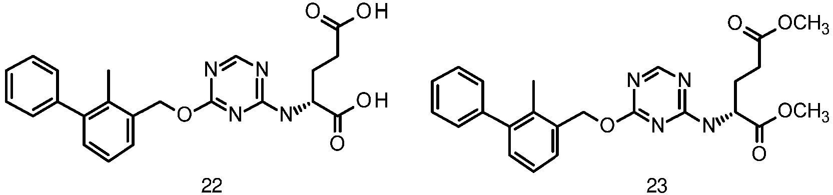

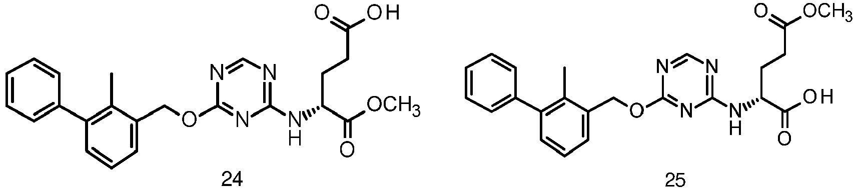

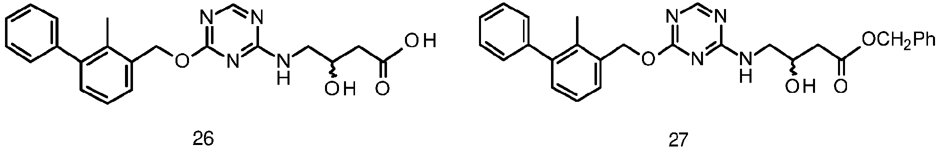

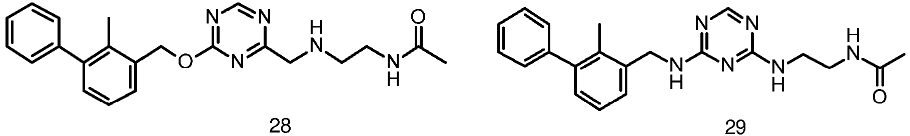

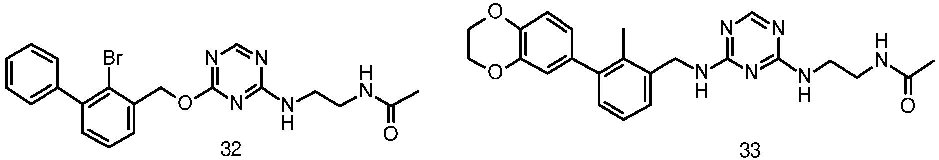

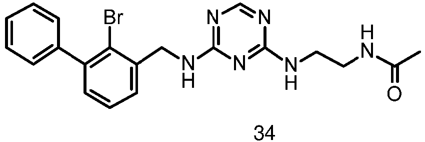

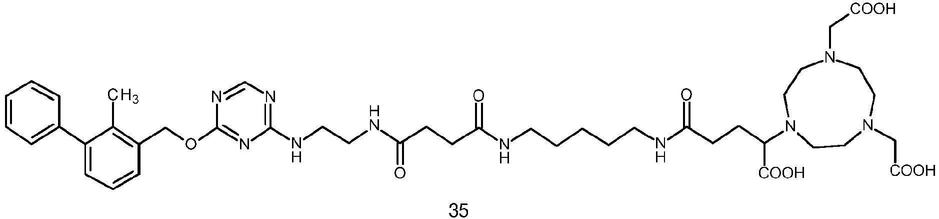

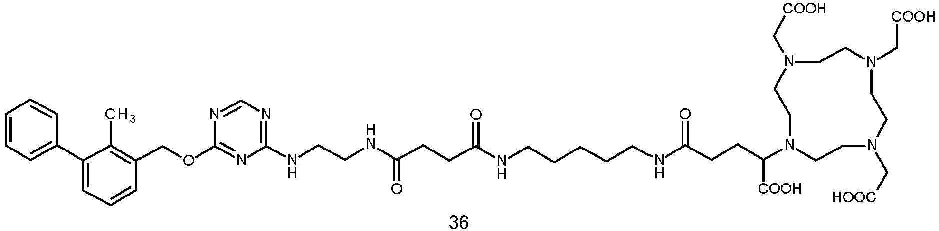

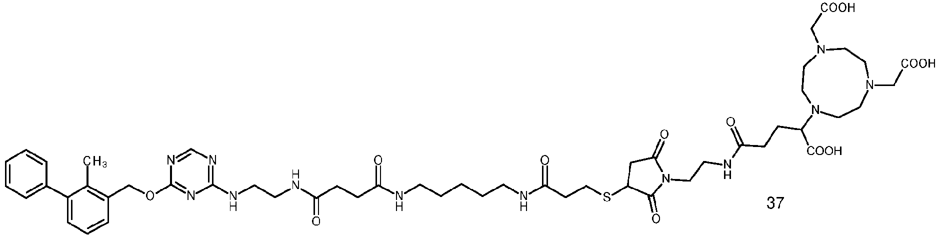

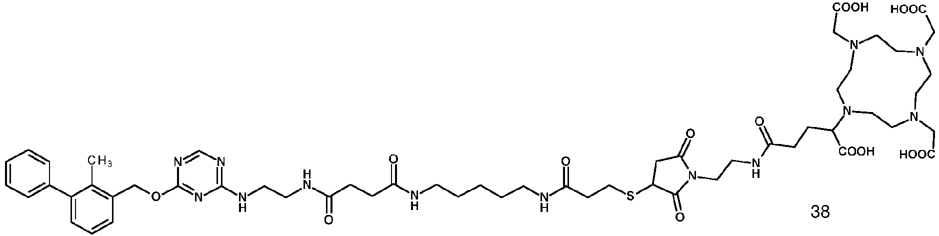

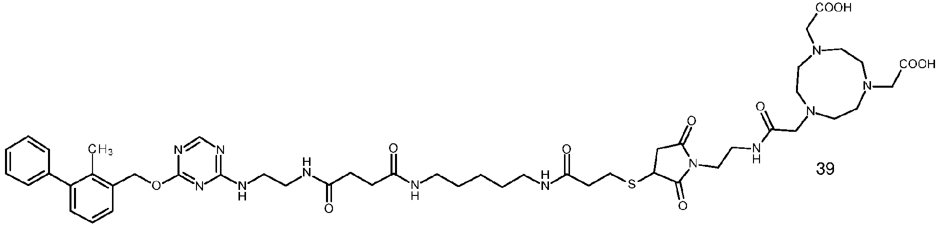

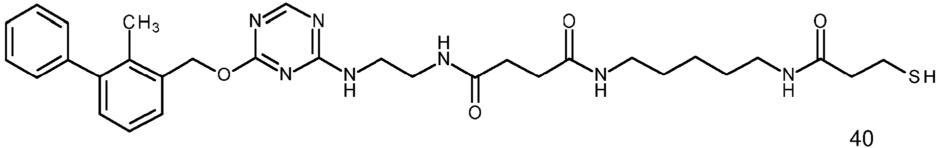

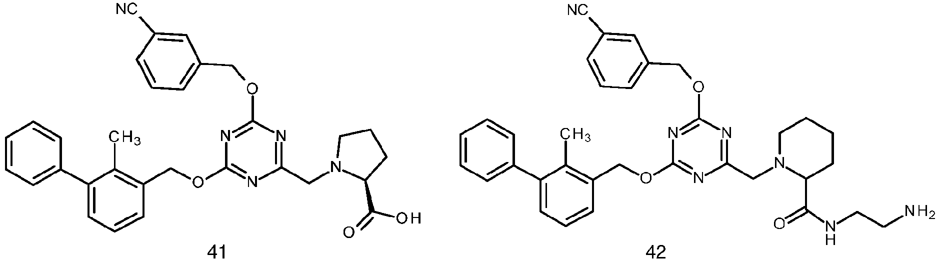

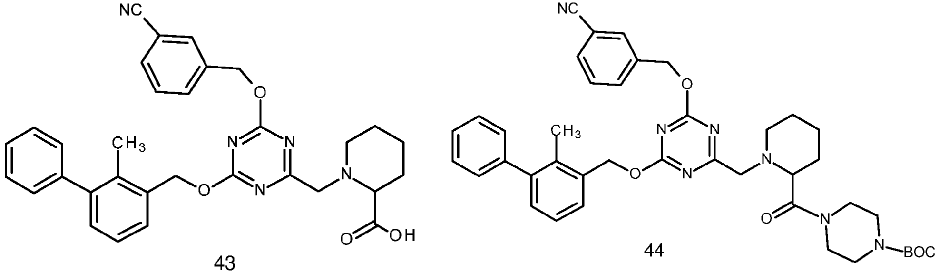

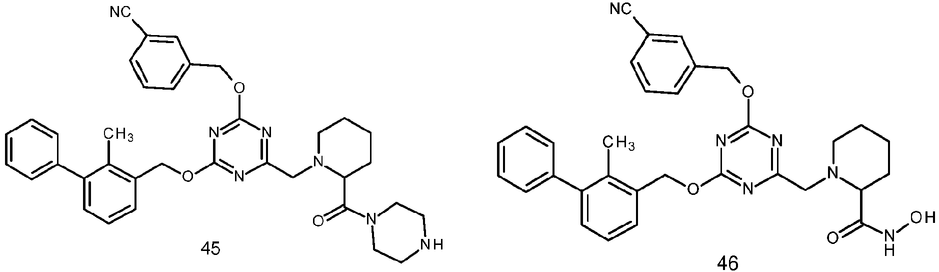

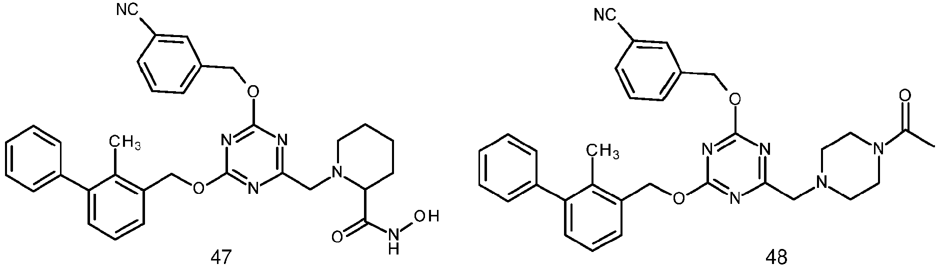

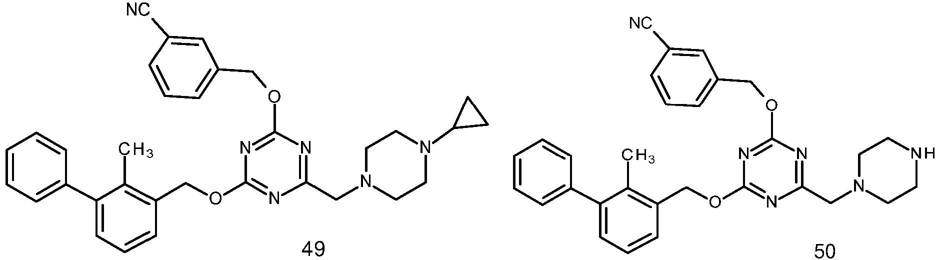

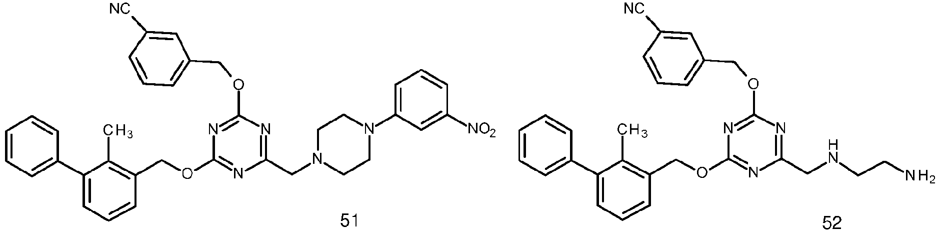

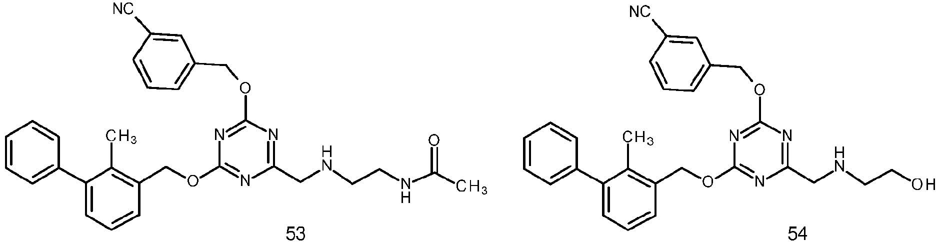

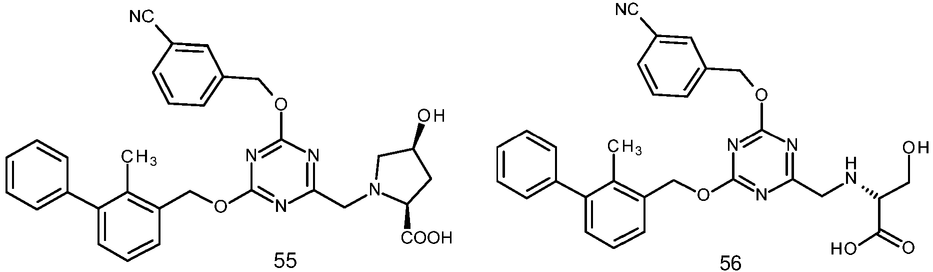

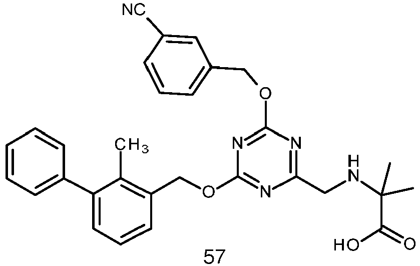

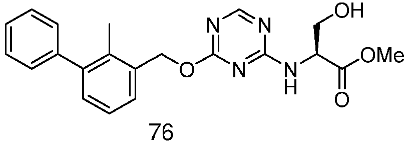

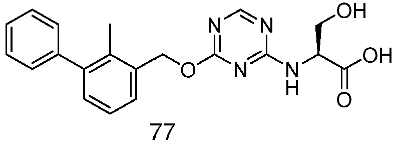

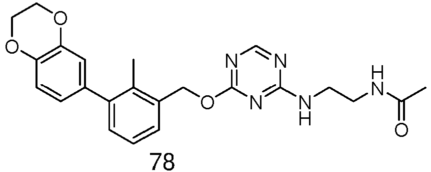

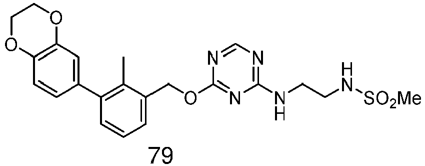

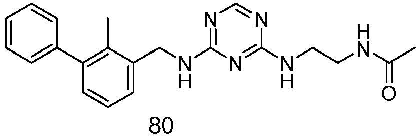

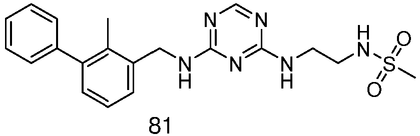

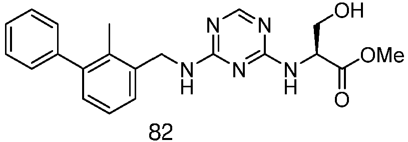

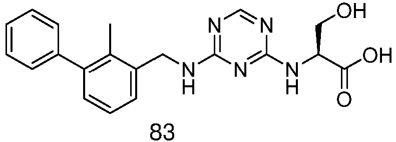

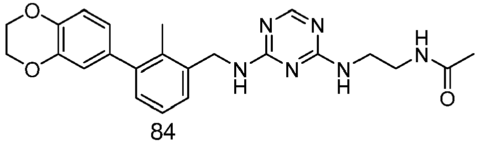

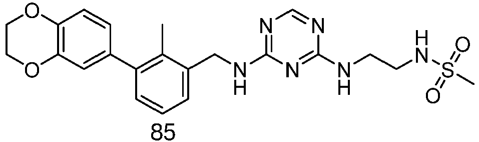

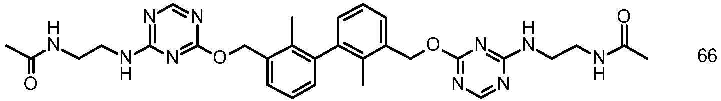

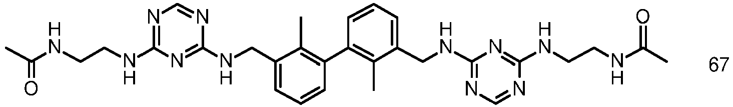

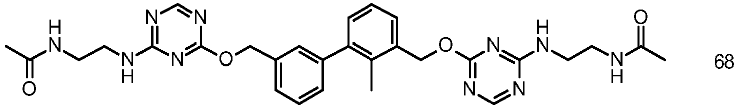

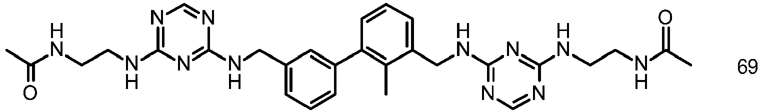

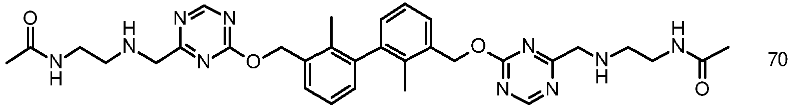

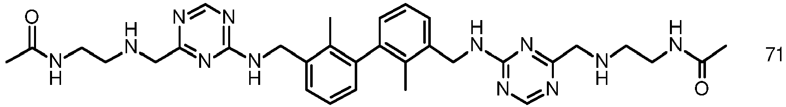

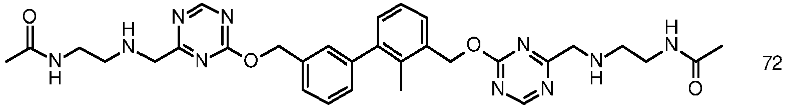

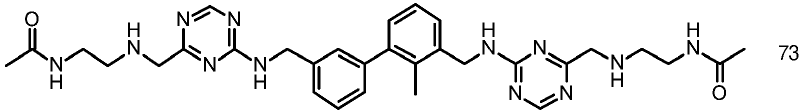

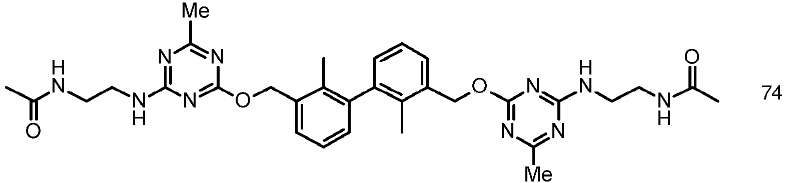

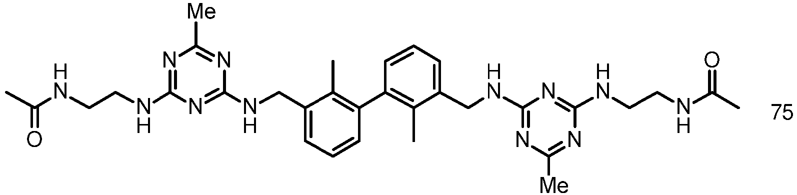

2. Pharmaceutical composition comprising a compound according to claim 1 and at least one pharmaceutically acceptable excipient.

3. Compound according to claim 1 for the use as a medicament.

4. Compound according to claim 1 for the use in the treatment or the prevention of a tumour, a viral infection, a bacterial infection or a neurodegenerative disease.

5. Compound for the use according to claim 4 **characterized in that** said tumour is selected from the group consisting of pancreatic cancer, bladder cancer, colorectal cancer, breast cancer, prostate cancer, renal cancer, hepatocellular cancer, lung cancer, ovarian cancer, cervical cancer, gastric cancer, oesophageal cancer, head and neck cancer, melanoma, neuroendocrine cancer, central nervous system cancer, brain cancer, bone cancer, soft tissue sarcoma, non-small cell lung cancer, small cell lung cancer or colon cancer, lymphocytic leukaemia, acute myeloid leukaemia, chronic lymphocytic leukaemia, small lymphocytic lymphoma, myelodysplastic syndrome, myeloproliferative disease, chronic myeloid leukaemia, multiple myeloma, non-Hodgkin's lymphoma, mantle cell lymphoma, follicular lymphoma, Waldestrom's macroglobulinemia, T-cell lymphoma, B-cell lymphoma or diffuse large B-cell lymphoma.

6. Compound for the use according to claim 4 **characterized in that** said neurodegenerative disease is selected from the group consisting of schizophrenia, Alzheimer's disease, multiple sclerosis, Parkinson's disease, Huntington's chorea, spinocerebellar ataxia type 1, amyotrophic lateral sclerosis, Batten disease.

7. Compound for the use according to claim 4 **characterized in that** said viral infection is caused by a virus selected from the group consisting of HIV, HBV, hepatitis A, B, C, D, papillomavirus herpes virus, influenza, COVID-19.

8. Combination therapy comprising a compound according to claim 1 and at least one additional anti-cancer agent selected from the group consisting of rituxan, doxorubicin, gemcitabine, nivolumab, pembrolizumab, atezolizumab, and nivolumab, pembrolizumab, atezolizumab, or ipilimumab or radiotherapy, and resection therapy.

9. Combination therapy comprising a compound according to claim 1 and a checkpoint inhibitor of a monoclonal antibody or antigen-binding fraction thereof and an additional therapeutic agent.

## Patentansprüche

1. Verbindung, ausgewählt aus der Gruppe, bestehend aus:

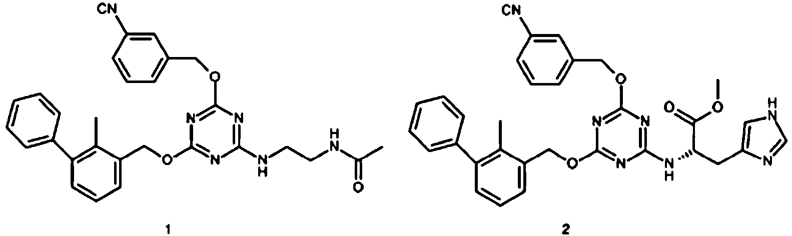

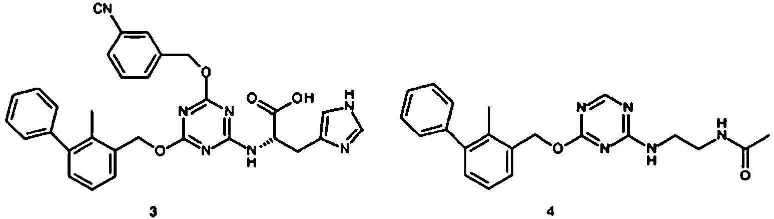

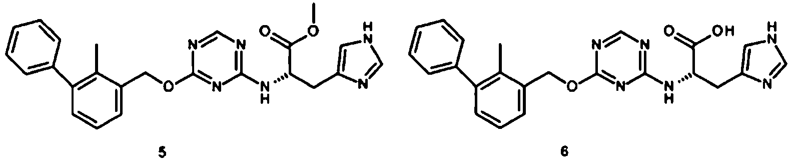

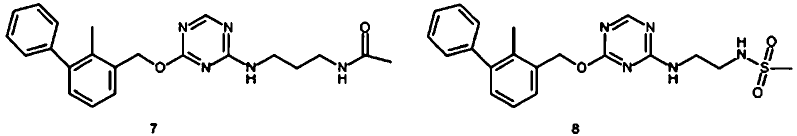

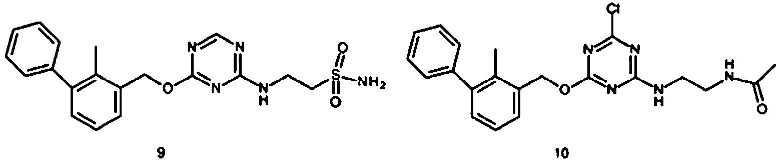

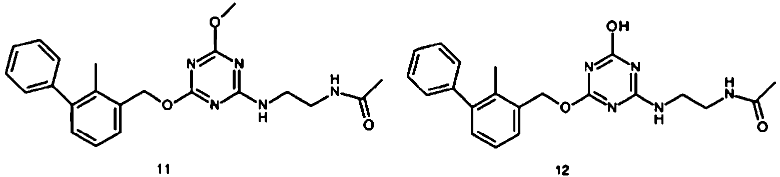

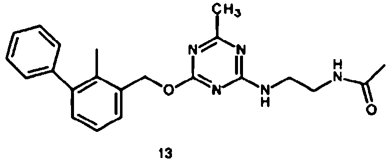

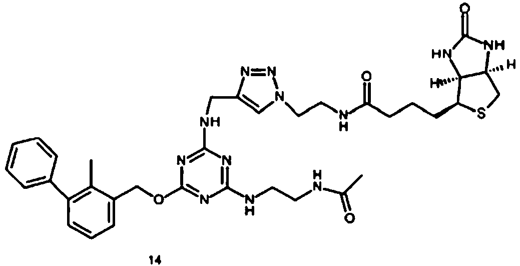

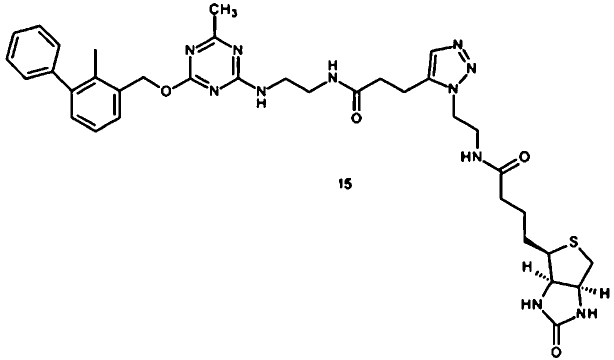

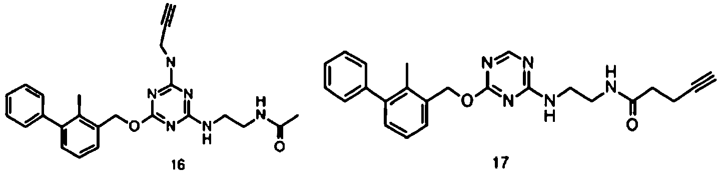

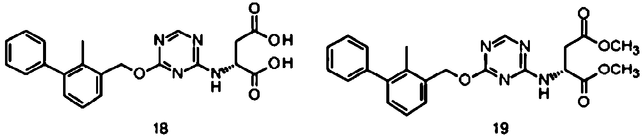

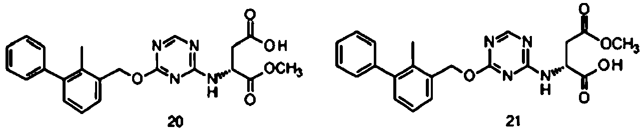

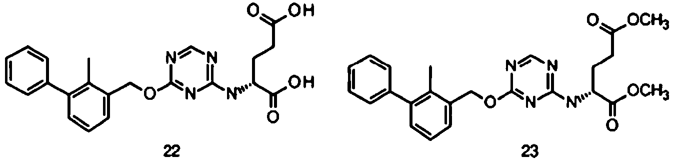

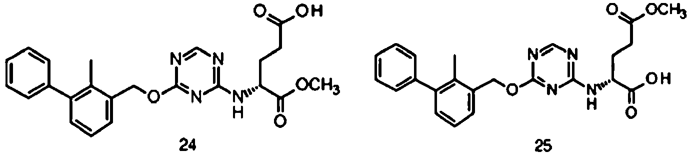

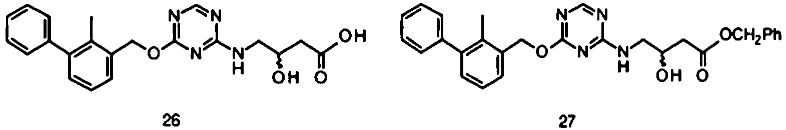

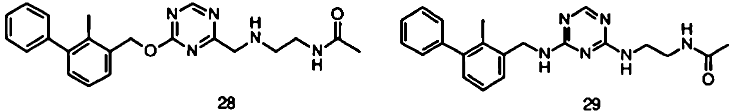

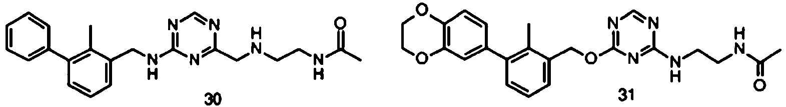

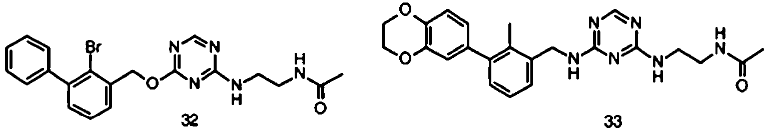

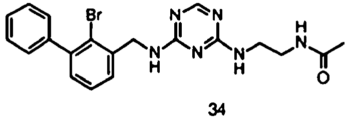

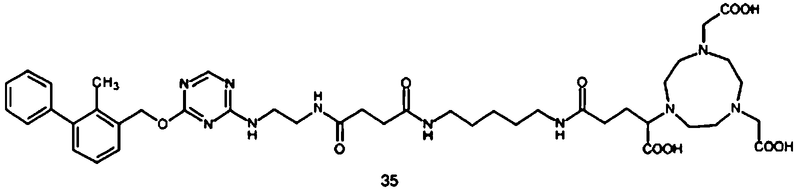

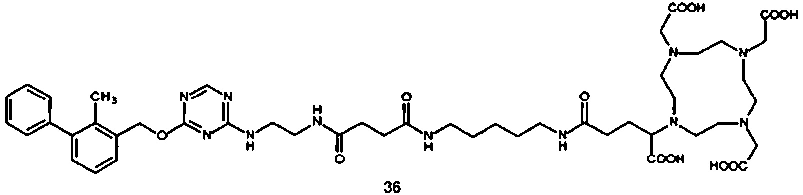

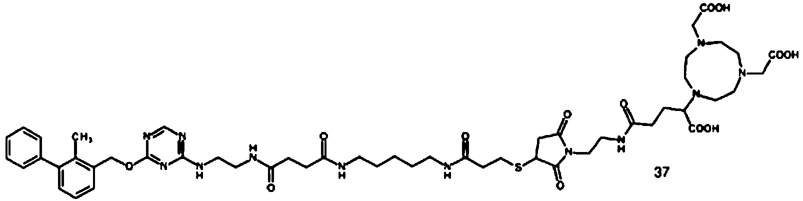

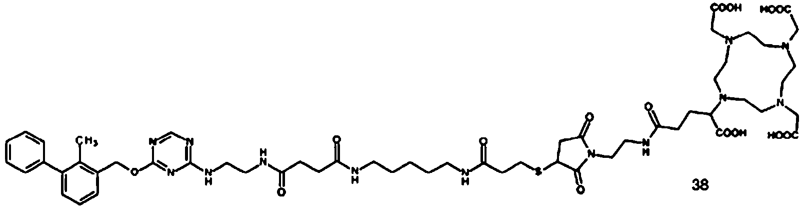

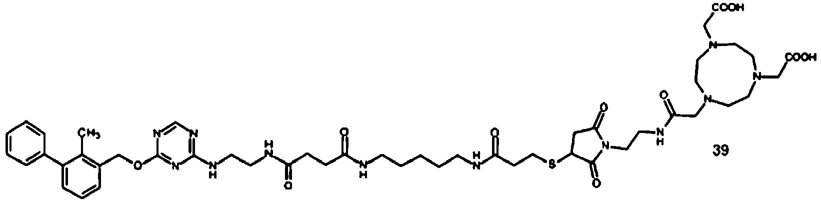

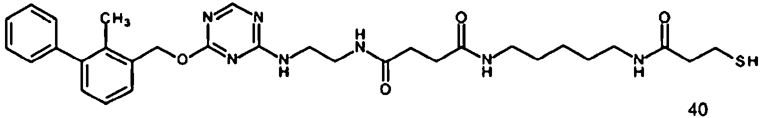

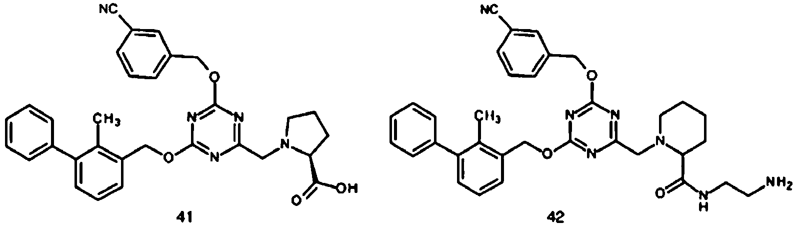

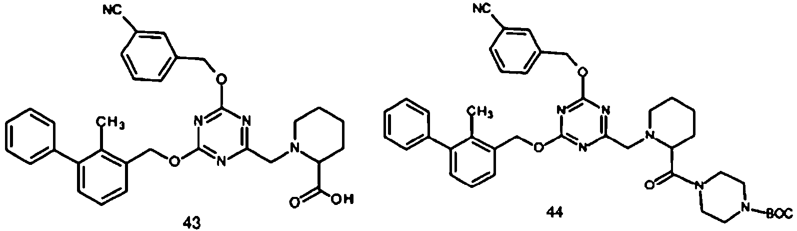

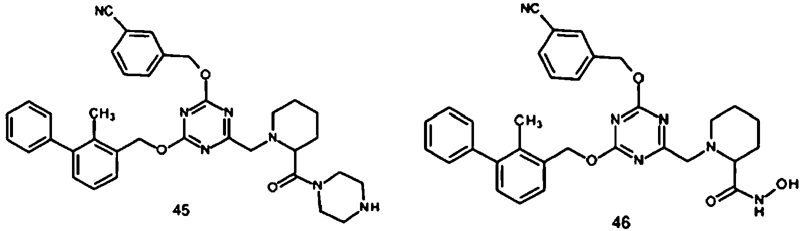

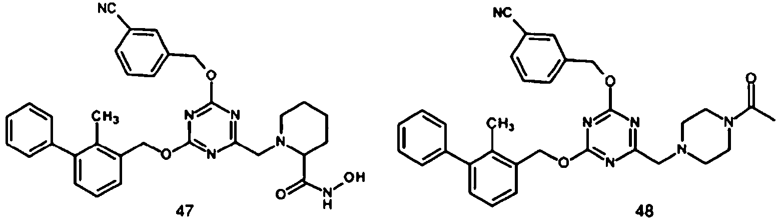

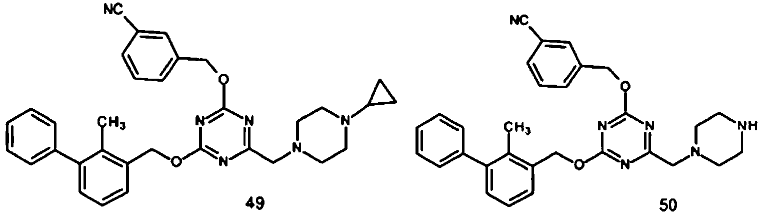

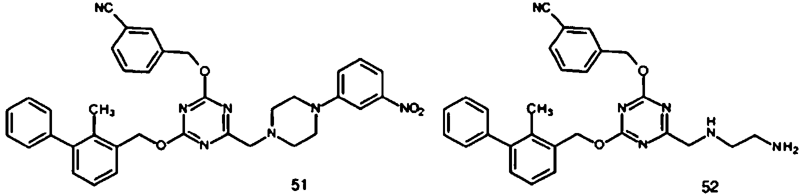

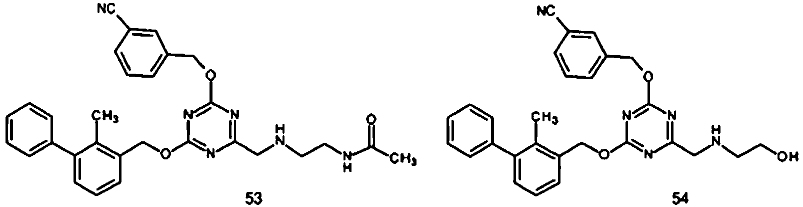

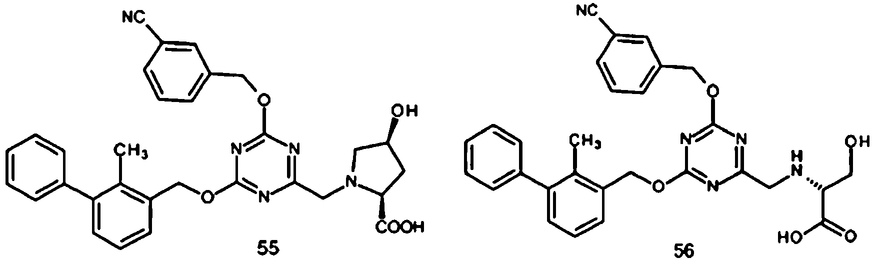

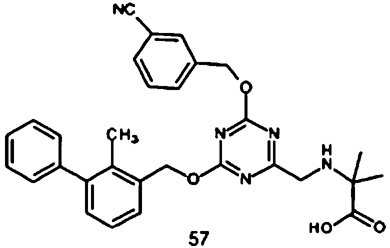

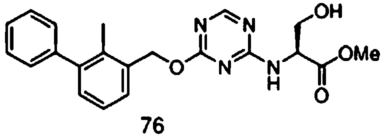

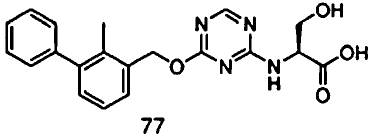

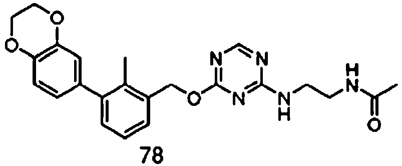

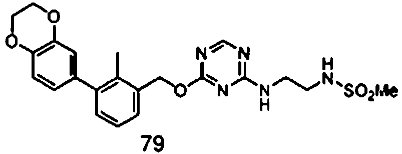

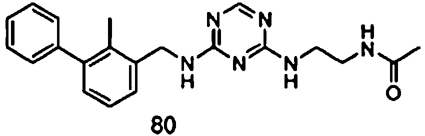

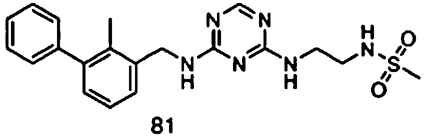

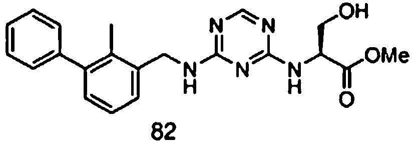

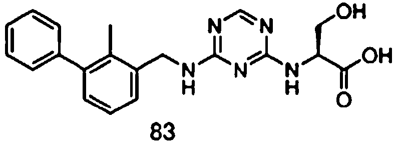

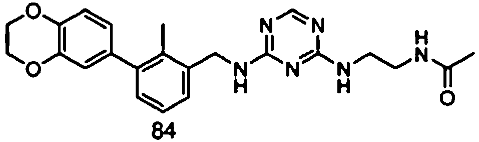

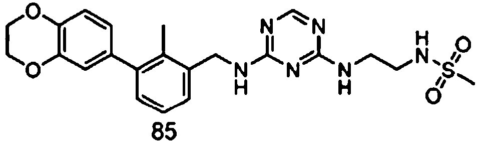

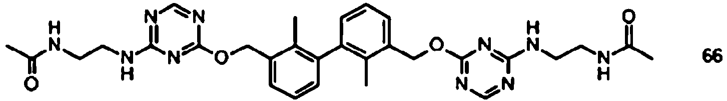

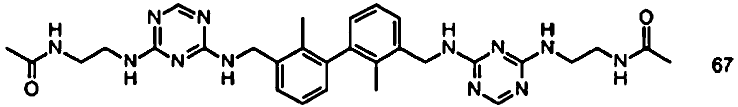

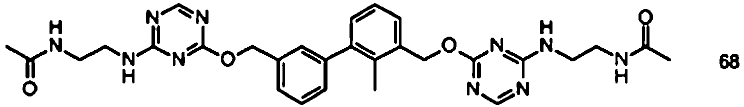

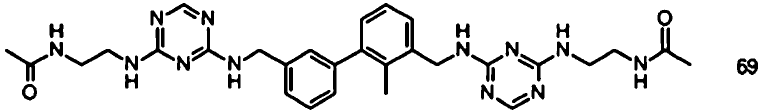

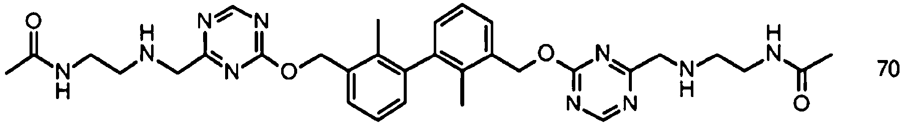

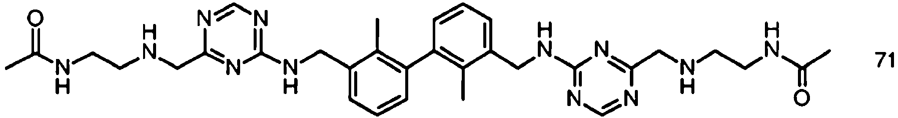

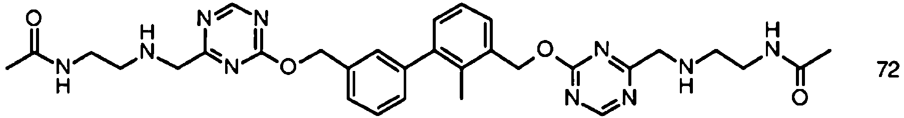

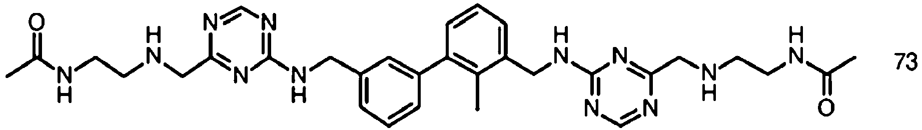

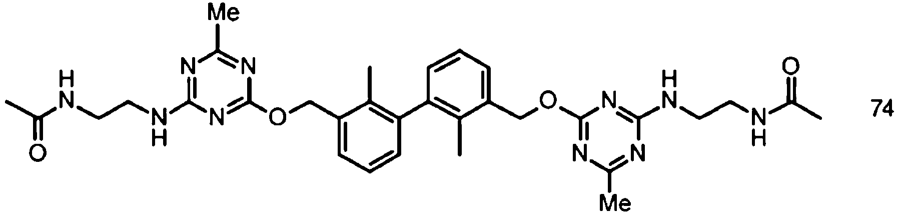

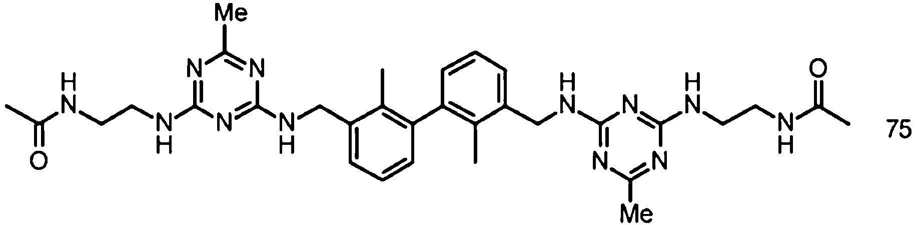

2. Pharmazeutische Zusammensetzung, umfassend eine Verbindung nach Anspruch 1 und mindestens einen pharmazeutisch akzeptablen Hilfsstoff.

3. Verbindung nach Anspruch 1 zur Verwendung als Arzneimittel.

4. Verbindung nach Anspruch 1 zur Verwendung bei der Behandlung oder Vorbeugung eines Tumors, einer Virusinfektion, einer bakteriellen Infektion oder einer neurodegenerativen Erkrankung.

5. Verbindung zur Verwendung nach Anspruch 4, **dadurch gekennzeichnet, dass** der Tumor ausgewählt ist aus der Gruppe, bestehend aus Bauchspeicheldrüsenkrebs, Blasenkrebs, Darmkrebs, Brustkrebs, Prostatakrebs, Nierenkrebs, Leberzellkrebs, Lungenkrebs, Eierstockkrebs, Gebärmutterhalskrebs, Magenkrebs, Speiseröhrenkrebs, Kopf- und Halskrebs, Melanom, neuroendokrinem Krebs, Krebs des zentralen Nervensystems, Hirnkrebs, Knochenkrebs, Weichteilsarkom, nichtkleinzelligem Lungenkrebs, kleinzelligem Lungenkrebs oder Dickdarmkrebs, lymphatischer Leukämie, akuter myeloischer Leukämie, chronischer lymphatischer Leukämie, kleinem lymphozytischem Lymphom, myelodysplastischem Syndrom, myeloproliferativer Erkrankung, chronischer myeloischer Leukämie, multiplem Myelom, Non-Hodgkin-Lymphom, Mantelzell-Lymphom, follikulärem Lymphom, Waldestrom-Makroglobulinämie, T-Zell-Lymphom, B-Zell-Lymphom oder diffusem großzelligem B-Zell-Lymphom.

6. Verbindung zur Verwendung nach Anspruch 4, **dadurch gekennzeichnet, dass** die neurodegenerative Erkrankung ausgewählt ist aus der Gruppe, bestehend aus Schizophrenie, Alzheimer-Krankheit, Multipler Sklerose, Parkinson-Krankheit, Chorea Huntington, spinozerebellärer Ataxie Typ 1, amyotropher Lateralsklerose, Batten-Krankheit.

7. Verbindung zur Verwendung nach Anspruch 4, **dadurch gekennzeichnet, dass** die Virusinfektion durch ein Virus, ausgewählt aus der Gruppe, bestehend aus HIV, HBV, Hepatitis A, B, C, D, Papillomavirus, Herpesvirus, Influenza, COVID-19, verursacht ist.

8. Kombinationstherapie, umfassend eine Verbindung nach Anspruch 1 und mindestens ein zusätzliches Antikrebsmittel, ausgewählt aus der Gruppe, bestehend aus Rituxan, Doxorubicin, Gemcitabin, Nivolumab, Pembrolizumab, Atezolizumab und Nivolumab, Pembrolizumab, Atezolizumab oder Ipilimumab oder Strahlentherapie und Resektionstherapie.

9. Kombinationstherapie, umfassend eine Verbindung nach Anspruch 1 und einen Checkpoint-Inhibitor eines monoklonalen Antikörpers oder einer antigenbindenden Fraktion davon und ein zusätzliches therapeutisches Mittel.

## Revendications

1. Composé choisi dans le groupe constitué par :

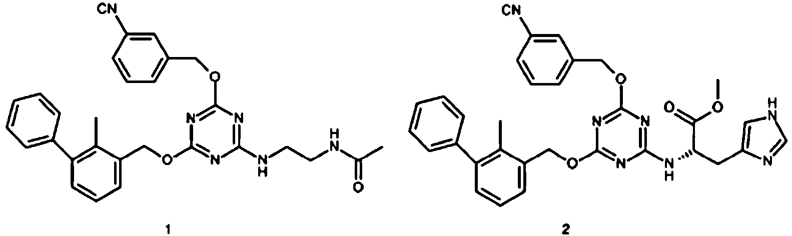

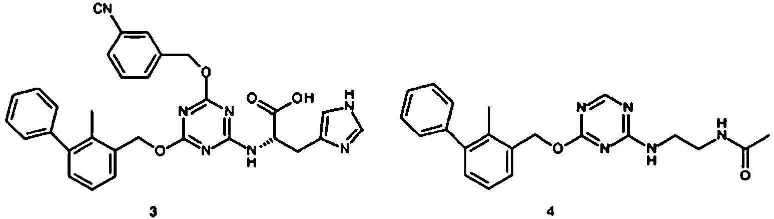

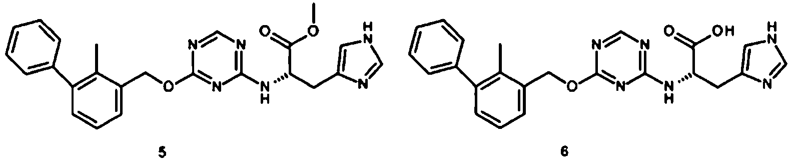

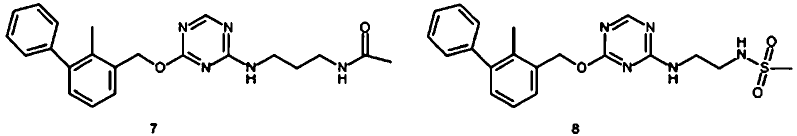

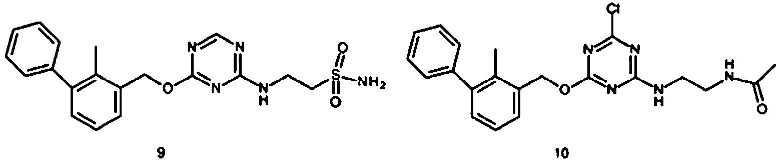

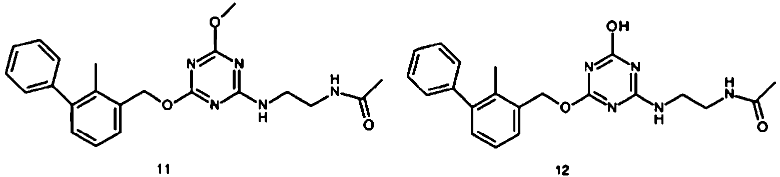

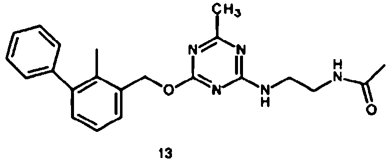

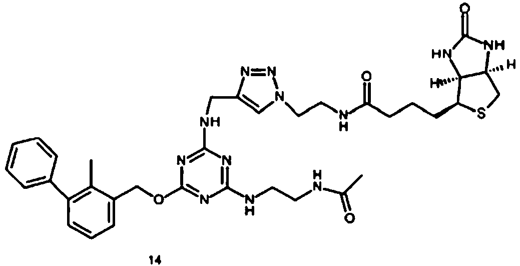

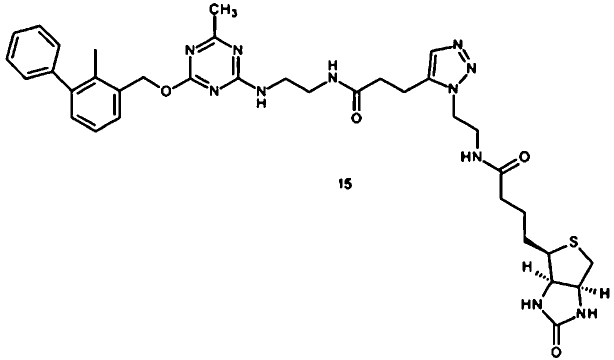

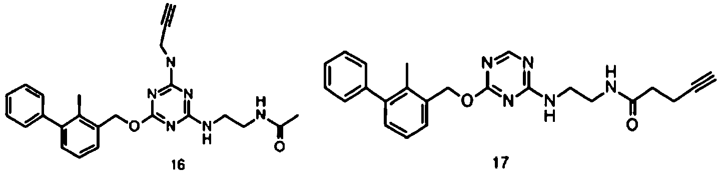

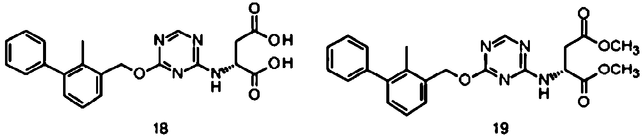

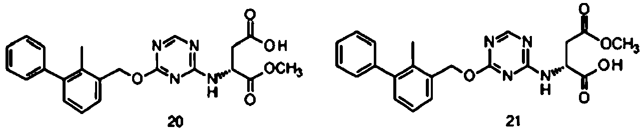

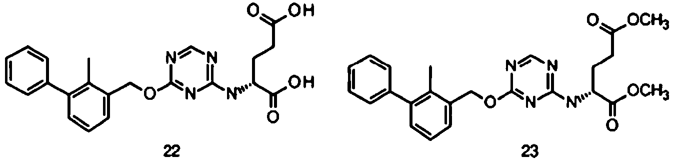

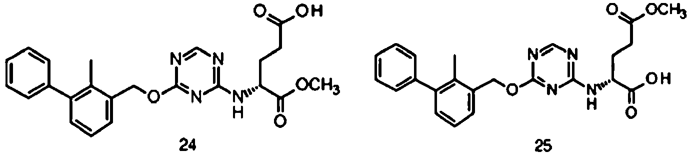

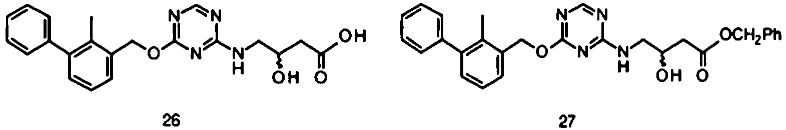

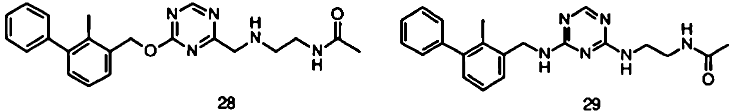

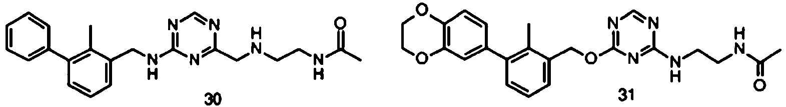

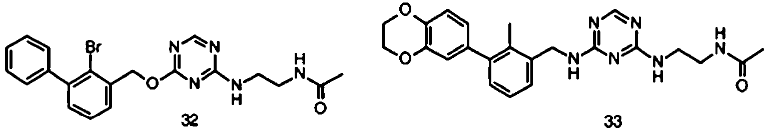

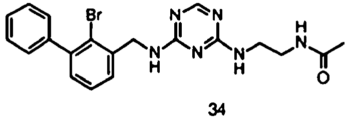

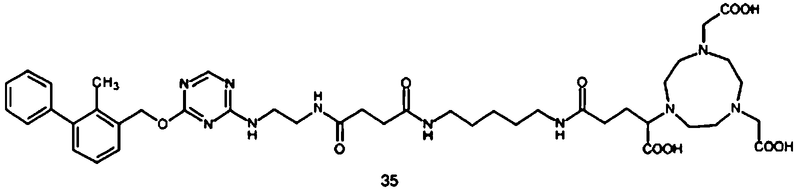

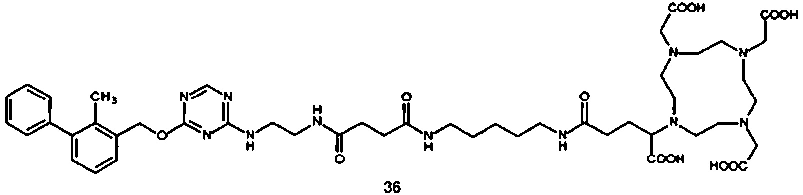

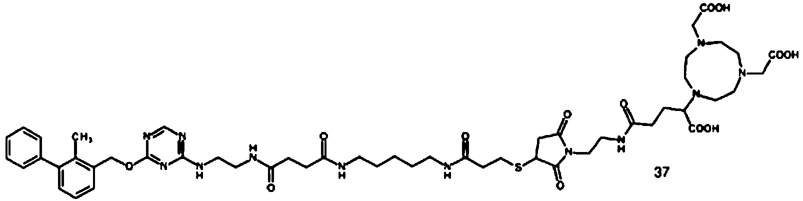

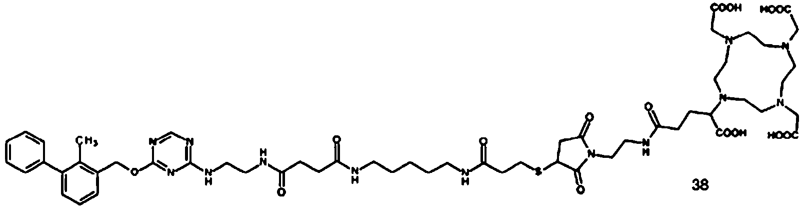

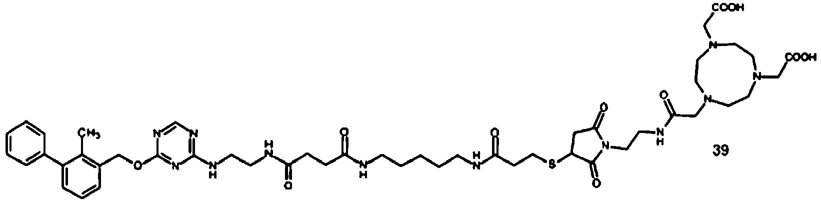

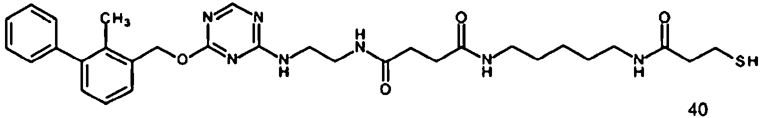

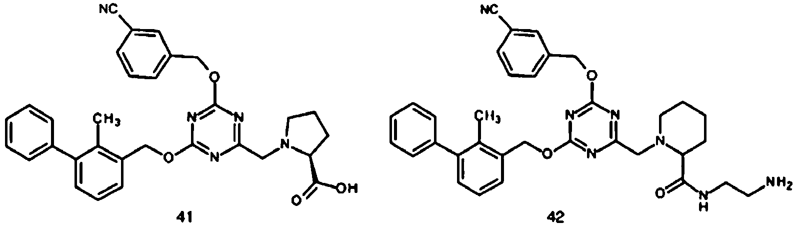

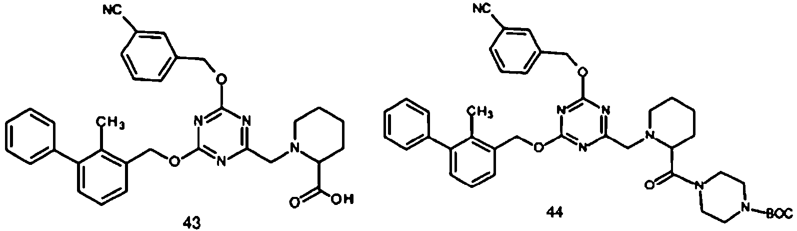

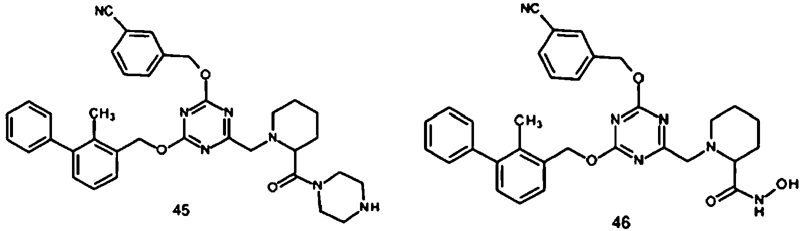

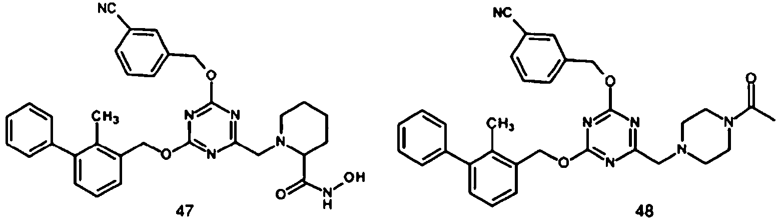

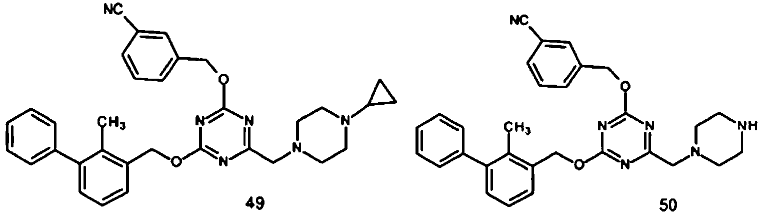

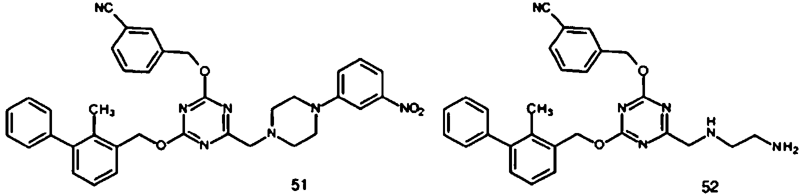

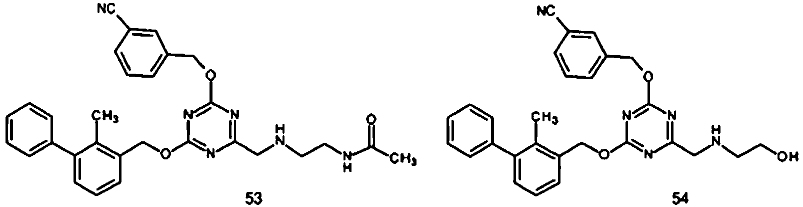

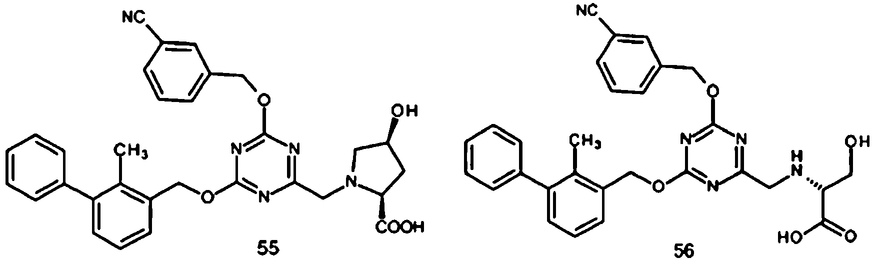

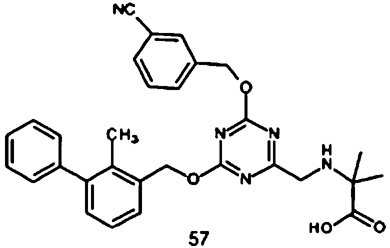

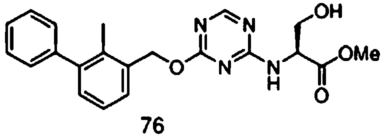

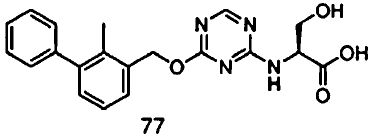

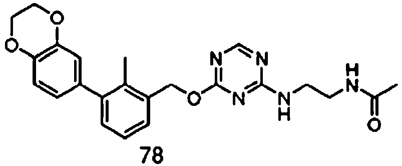

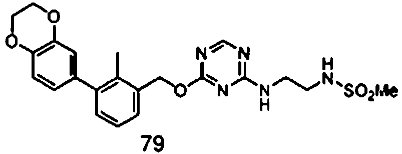

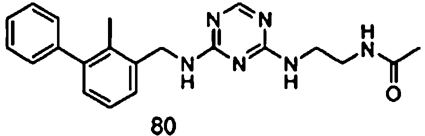

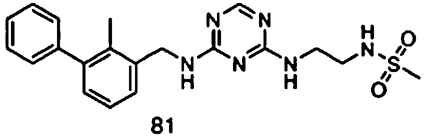

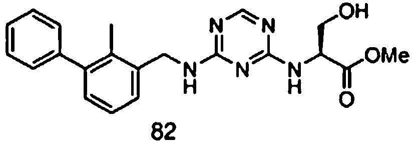

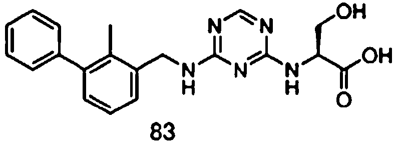

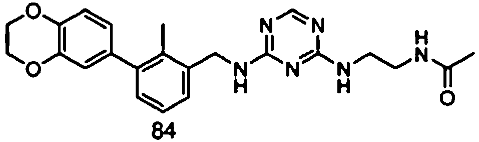

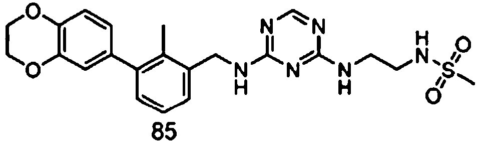

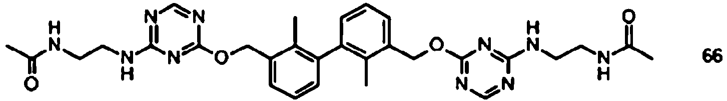

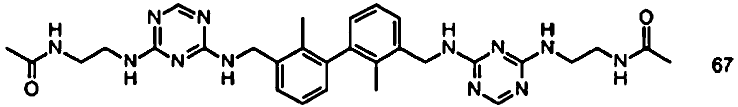

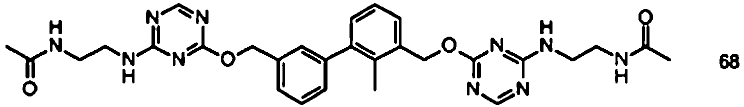

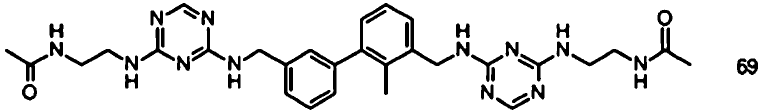

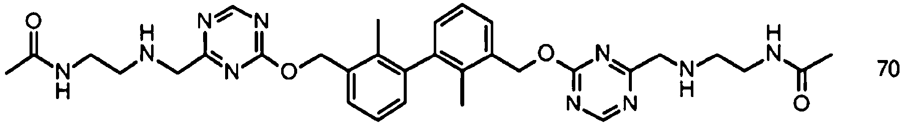

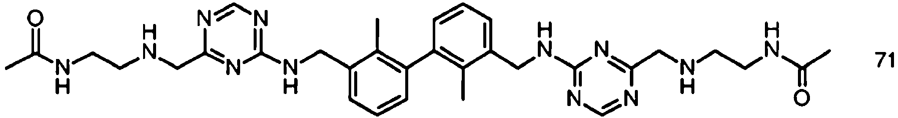

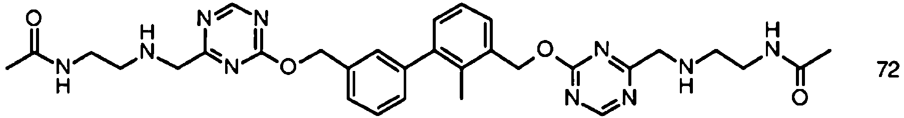

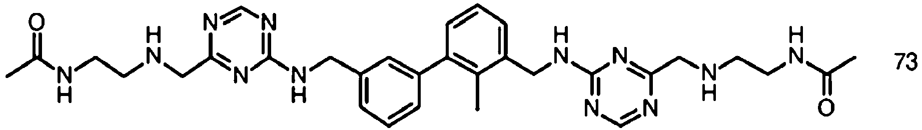

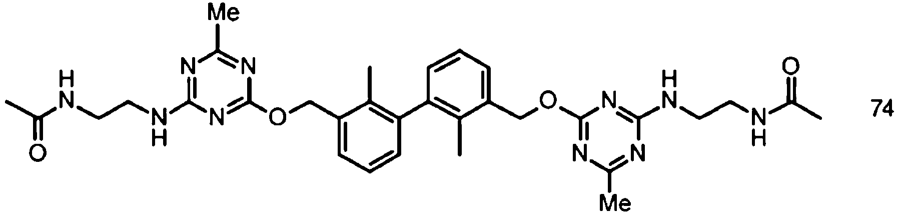

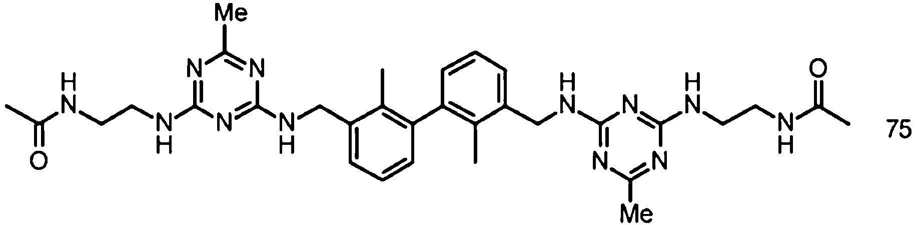

2. Composition pharmaceutique comprenant un composé selon la revendication 1 et au moins un excipient pharmaceutiquement acceptable.

3. Composé selon la revendication 1, destiné à être utilisé en tant que médicament.

4. Composé selon la revendication 1, destiné à être utilisé dans le traitement ou la prévention d'une tumeur, d'une infection virale, d'une infection bactérienne ou d'une maladie neurodégénérative.

5. Composé destiné à être utilisé selon la revendication 4, **caractérisé en ce que** ladite tumeur est choisie dans le groupe constitué par le cancer du pancréas, le cancer de la vessie, le cancer colorectal, le cancer du sein, le cancer de la prostate, le cancer du rein, le cancer hépatocellulaire, le cancer du poumon, le cancer des ovaires, le cancer du col de l'utérus, le cancer de l'estomac, le cancer de l'œsophage, le cancer de la tête et du cou, le mélanome, le cancer neuroendocrinien, le cancer du système nerveux central, le cancer du cerveau, le cancer des os, le sarcome des tissus mous, le cancer du poumon non à petites cellules, le cancer du poumon à petites cellules ou le cancer du côlon, la leucémie lymphocytaire, la leucémie myéloïde aiguë, la leucémie lymphocytaire chronique, le lymphome lymphocytaire de petite taille, le syndrome myélodysplasique, la maladie myéloproliférative, la leucémie myéloïde chronique, le myélome multiple, le lymphome non hodgkinien, lymphome à cellules du manteau, le lymphome folliculaire, la macroglobulinémie de Waldestrom, le lymphome à cellules T, le lymphome à cellules B ou lymphome diffus à grandes cellules B.

6. Composé destiné à être utilisé selon la revendication 4, **caractérisé en ce que** ladite maladie neurodégénérative est choisie dans le groupe constitué par la schizophrénie, la maladie d'Alzheimer, la sclérose en plaques, la maladie de Parkinson, la chorée de Huntington, l'ataxie spinocérébelleuse de type 1, la sclérose latérale amyotrophique, la maladie de Batten.

7. Composé destiné à être utilisé selon la revendication 4, **caractérisé en ce que** l'infection virale est causée par un virus choisi dans le groupe constitué par le VIH, le VHB, l'hépatite A, B, C, D, le papillomavirus, le virus de l'herpès, la grippe, le COVID-19.

8. Traitement combiné comprenant un composé selon la revendication 1 et au moins un agent anticancéreux supplémentaire choisi dans le groupe constitué par le rituxan, la doxorubicine, la gemcitabine, le nivolumab, le pembrolizumab, l'atezolizumab et le nivolumab, le pembrolizumab, l'atezolizumab ou l'ipilimumab, ou par la radiothérapie et la thérapie de résection.

9. Traitement combiné comprenant un composé selon la revendication 1 et un inhibiteur de point de contrôle d'un anticorps monoclonal ou d'une fraction de liaison à l'antigène de celui-ci et un agent thérapeutique supplémentaire.
